(19) Europäisches Patentamt
European Patent Office
Office européen des brevets

(11) Publication number: **0 304 057 B1**

(12) # EUROPEAN PATENT SPECIFICATION

(45) Date of publication of patent specification: **20.04.94**

(51) Int. Cl.5: **C07D 213/68**, C07D 213/89, C07D 401/04, C07D 405/06, C07D 405/04, C07D 409/04, C07D 409/06, C07D 403/04, A01N 43/40, A01N 43/56

(21) Application number: **88113434.0**

(22) Date of filing: **18.08.88**

(54) **Pyridinone derivatives, and agricultural and horticultural fungicidal compositions containing the same.**

(30) Priority: **18.08.87 JP 204375/87**
**18.08.87 JP 204376/87**
**09.10.87 JP 255735/87**
**15.10.87 JP 260473/87**
**30.07.88 JP 191636/88**
**30.07.88 JP 191637/88**
**06.08.88 JP 196316/88**

(43) Date of publication of application:
**22.02.89 Bulletin 89/08**

(45) Publication of the grant of the patent:
**20.04.94 Bulletin 94/16**

(84) Designated Contracting States:
**BE CH DE FR GB IT LI NL**

(56) References cited:

**CHEMICAL ABSTRACTS, vol. 107, 1987, page 315, abstract no. 231422h, Columbus, Ohio, US**

(73) Proprietor: **KUMIAI CHEMICAL INDUSTRY CO., LTD.**
**4-26, Ikenohata 1-chome**
**Taitoh-ku Tokyo 110(JP)**

Proprietor: **IHARA CHEMICAL INDUSTRY Co., Ltd.**
**4-26, Ikenohata 1-chome**
**Taitoh-ku, Tokyo 110(JP)**

(72) Inventor: **Nezu, Yukio**
**6-12, Nyakuoji 2-chome**
**Fujieda-shi Shizuoka-ken(JP)**
Inventor: **Yonekura, Norihisa**
**132, Kojima**
**Fukude-cho**
**Iwata-gun Shizuoka-ken(JP)**
Inventor: **Takehi, Takayoshi**
**132, Kojima**
**Fukude-cho**
**Iwata-gun Shizuoka-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 304 057 B1

CHEMICAL ABSTRACTS, vol. 73, 1970, page 304, abstract no. 45305b, Columbus, Ohio, US; T. KATO et al.: "Ketene and its derivatives. 37. Reaction of diketene with ketone anils and related compounds"

CHEMICAL ABSTRACTS, vol. 110, 1989, page 722, abstract no. 192549v, Columbus, Ohio, US; A.M. ZVONOK et al.: "Reaction of (beta-arylacryloyl)oxiranes with phenyl azide. Synthesis and reactions of (beta-anilino-beta-arylacryloyl)oxiranes"

Inventor: **Yokota, Sumio**
**132, Kojima**
**Fukude-cho**
**Iwata-gun Shizuoka-ken(JP)**
Inventor: **Kojima, Yoshiyuki**
**69, Kougosho**
**Kakegawa-shi Shizuoka-ken(JP)**
Inventor: **Yaguchi, Shigeharu**
**12-3, Honmachi 6-chome**
**Yaizu-shi Shizuoka-ken(JP)**
Inventor: **Maeno, Shinichiro**
**1809, Kamo**
**Kikugawa-cho**
**Ogasa-gun Shizuoka-ken(JP)**
Inventor: **Muramatsu, Norimichi**
**15-11, Katsuragaoka 3-chome**
**Kakegawa-shi Shizuoka-ken(JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

## Description

### Field of the Invention

This invention relates to new 4(1H)-pyridinone derivatives which are useful as new compounds or agents having improved fungicidal activities. This invention also relates to an agricultural or horticultural fungicidal composition containing the new 4(1H)-pyridinone derivatives as active ingredient. This invention further relates to processes for the production of the new 4(1H)-pyridinone derivatives.

### Prior Art

In Japanese Patent Application first publication "Kokai" No. 181204/87, we, the present applicants disclosed that 1,2,6-triphenyl-4(1H)-pyridinone derivatives show broad antibacterial and antifungal spectra against pathogenic microorganisms of various, agricultural and horticultural diseases of plants, such as rice sheath blight (Rhizoctonia solani), rice blast (Pyricularia oryzae), cucumber downy mildew (Pseudoperonospora cubensis), cucumber powdery mildew (Sphaerotheca fuliginea) and Alternaria sooty spot of chinese mustard (Alternaria brassicicola). On the other hand, Japanese Patent Application first publication "Kokai" No. 65871/81 discloses 1-substituted phenyl-2,6-dimethyl-4(1H)-pyridinone derivatives. It also contains a disclosure to the effect that these derivatives are not only effective for cucumber powdery mildew (Sphaerotheca fuliginea) but also useful as slime control agents. Similarly, Japanese Patent Application first publication "Kokai" No. 102504/80 discloses 2,6-diphenyl-4(1H)-pyridinone and contains a description to the effect that this compound is effective for rice blast (Pyricularia oryzae) and cucumber anthracnose (Colletotrichum lagenarium). An article of the "Synthetic Communications" 13(5), 411-417 (1983) also describes a process for the production of 1,2,6-triphenyl-substituted 4(1H)-pyridinone derivatives by reactions between ketimines and ethyl phenylpropionate, and this article discloses some examples of the 1,2,6-triphenyl-substituted 4(1H)-pyridinone derivatives produced, which are merely described there to be interesting as intermediate compounds for synthetic production of other, final products. On the other hand, Japanese journal "Yakugaku Zasshi" 90(5), 613-617 (1970) describes a process for the production of 4(1H)-pyridinone derivatives by reactions between diketene and imines, and this article discloses some examples of the 4(1H)-pyridinone derivatives produced.

Although the compounds disclosed in Japanese Patent Application first publication "Kokai" No. 181204/87 are effective in controlling rice sheath blight (Rhizoctonia solani), the compounds do not always exhibit satisfactory activity to control other diseases disclosed above. In addition, the above article in the "Synthetic Communications" and Japanese journal "Yakugaku Zasshi" do not describe anything about the biological activities of the compounds disclosed therein.

In order to provide a novel, agricultural and horticultural fungicide more useful than the known compounds described in the above-mentioned publications, we, the present inventors, have paid our attention on the 4(1H)-pyridinone derivatives and its skeleton structure and have carried out extensive investigations to provide their new 3-substituted derivatives. As a result, we have now succeeded to synthesize new compounds of this invention. It has also been found that the new compounds of this invention have improved and excellent activities as agricultural and horticultural fungicides, leading to completion of this invention.

### DETAILED DESCRIPTION OF THE INVENTION

According to a first and broadest aspect of this invention, there are provided as new compounds 4(1H)-pyridinone derivatives represented by the general formula:

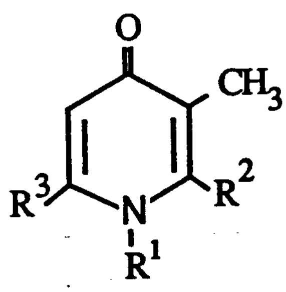

(I)

wherein $R^1$ represents a cycloalkyl group having 3-6 carbon atoms; a pyridyl group which may be substituted with an alkoxy group having 1-6 carbon atoms or a halogen atom; a morpholino group; a pyrrolidino group; a piperidino group; a phenyl group which may be substituted with a halogen atom, a lower alkyl group having 1-6 carbon atoms, a lower alkoxy group having 1-6 carbon atoms, a trifluoromethyl group or a nitro group; a benzyl group which may be substituted with a halogen atom;

$R^2$ represents a hydrogen atom; an alkyl group having 1-10 carbon atoms; a cycloalkyl group having 3-6 carbon atoms; a phenyl group which may be substituted with a halogen atom; a naphthyl group; a pyridyl group which may be substituted with a lower alkoxy group having 1-6 carbon atoms; a thienyl group which may be substituted with a lower alkyl group having 1-6 carbon atoms; a furyl group which may be substituted with a lower alkyl group having 1-6 carbon atoms; a pyridin-1-oxide group; an 1,3,5-trimethyl-pyrazol-4-yl group;

$R^3$ represents a hydrogen atom; an alkyl group having 2-10 carbon atoms; a cycloalkyl group having 3-6 carbon atoms; a cycloalkenyl group having 6 carbon atoms; a phenyl group which may be substituted with a halogen atom; a pyridyl group which may be substituted with a lower alkoxy group having 1-6 carbon atoms; a furyl group which may be substituted with a lower alkyl group having 1-6 carbon atoms; a naphthyl group; a thienyl group which may be substituted with a lower alkyl group having 1-6 carbon atoms; or a 3-thienylmethyl group;

with the proviso that $R^1$, $R^2$ and $R^3$ are not an unsubstituted or substituted phenyl group simultaneously.

The compound of the general formula (I) according to this invention may also be converted into their salts with an acid or a cation.

In the compound of the general formula (I) according to this invention, the alkyl group as represented by the substituents $R^1$, $R^2$, $R^3$, $X^1$, $X^2$, $X^3$, Y and Z, as well as the alkyl group occasionally present in these substituents and others are an alkyl group, containing 1 to 10 carbon atoms, and preferably an alkyl group containing 1 to 8 carbon atoms. When $R^3$ is an alkyl group, it contains at least 2 carbon atoms. The alkyl group may include straight chain or branched chain alkyl groups, and suitable examples of which include methyl, ethyl, n-propyl, isopropyl, n-butyl, isobutyl, tert-butyl, pentyl, hexyl and heptyl.

The lower alkyl group or the lower alkoxy group which can be used as a substituent on the substituents $R^1$, $R^2$, $R^3$, $X^1$, $X^2$, $X^3$, Y and Z may be an alkyl group or an alkoxy group, which may be straight chain or branched chain, having 1 to 6 carbon atoms, and preferably 1 to 4 carbon atoms. Typical examples of the lower alkoxy group and the lower alkoxy group may include methyl, methoxy and ethoxy.

The cycloalkyl group as represented by the substituents $R^1$, $R^2$ and $R^3$ may include cyclopropyl, cyclopentyl and cyclohexyl.

The cycloalkenyl group as represented by the substituents $R^3$ may include a cyclohexenyl group.

The halogen group includes bromine, chlorine, fluorine and iodine.

According to particular embodiments of the first aspect of this invention, there are provided the following four types of the compounds:

(A) A first group of typical examples of the compounds of this invention are those of the general formula (I),

in which $R^1$ represents a cycloalkyl group, a pyridyl group which may be substituted with a lower alkoxy group or a halogen atom, a morpholino group, a pyrrolidino group or a piperidino group;

$R^2$ represents a phenyl group;

$R^3$ represents a substituted or unsubstituted phenyl group of the formula

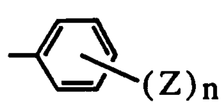

where Z is a halogen atom and n is an integer of 0 to 2.

$R^1$ may further represent an alkyl group, a pyridyl group which is substituted with a lower alkyl group, a perhydroazepin-1-yl group, a pyrimidyl group which may be substituted a halogen atom, a furylmethyl group.

(B) A second group of typical examples of the compounds of this invention are those of the general formula (I),

in which $R^1$ represents a substituted or unsubstituted phenyl group of the formula

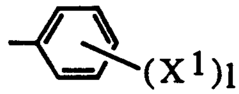

where $X^1$ is a halogen atom or a lower alkoxy group and $l$ is an integer of 0 to 2;

$R^2$ represents a phenyl group, a pyridin-1-oxide group or furyl group which may be substituted with a lower alkyl group;

$R^3$ represents a phenyl group, a pyridyl group which may be substituted with a lower alkoxy group or a furyl group which may be substituted with a lower alkoxy group.

Also, $l$ may be an integer of 3.

(C) A third group of typical examples of the compounds of this invention are those of the general formula (I),

in which $R^1$ represents a substituted or unsubstituted phenyl group of the formula

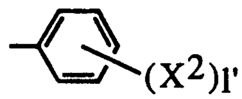

where $X^2$ is a halogen atom, a lower alkyl group, a lower alkoxy group, a trifluoromethyl group or a nitro group and $l'$ is an integer of 0 to 3;

$R^2$ represents an unsubstituted phenyl group;

$R^3$ represents a hydrogen atom or an alkyl group containing more than two carbon atoms.

$R^2$ may further represent a substituted phenyl group of the formula

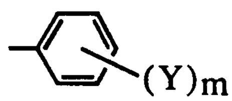

where Y represents a halogen atom and $m$ is an integer of 1;

(D) A fourth group of typical examples of the compounds of this invention are those of the general formula (I),

in which $R^1$ is a benzyl group which may be substituted with a halogen atom or a substituted or unsubstituted phenyl group of the formula

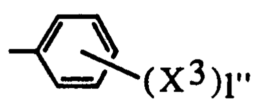

where $X^3$ is a halogen atom, a lower alkyl group, a lower alkoxy group, a trifluoromethyl group or a nitro group and $l''$ is an integer of 0 to 2;

$R^2$ represents a hydrogen atom, an alkyl group, a phenyl group which may be substituted with a halogen atom, a pyridyl group which may be substituted with a lower alkoxy group, a thienyl group which may be substituted with a lower alkyl group, a cycloalkyl group, a styryl group or a 1,3,5-trimethylpyrazol-4-yl group;

$R^3$ represents a thienyl group which may be substituted with a lower alkyl group, a phenyl group which may be substituted with a halogen atom, a cycloalkyl group, a naphtyl group or a 3-thienylmethyl group;

with the proviso that $R^1$, $R^2$ and $R^3$ are not an unsubstituted or substituted phenyl group simultaneously and that $R^2$ is an alkyl group or a cycloalkyl group when $R^3$ is an alkyl group.

$R^1$ may further represent a substituted phenyl group of the formula

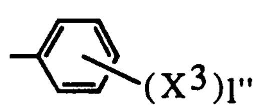

where $X^3$ is the same as defined above and $l''$ is an integer of 3.

$R^2$ may additionally represent an alkyl group which is substituted with a lower alkoxy group.

Those compounds of the general formula (I) in which two of $R^1$, $R^2$ and $R^3$ each represents an unsubstituted or substituted phenyl group are preferred. Also, those compounds of the general formula (I) in which $R^1$ represents a mono-, di- or tri-substituted phenyl group such as a halogen-substituted phenyl group or a methoxy-substituted phenyl group.

Another preferred compounds are those of the general formula (I) in which $R^1$ is a phenyl group and $R^2$ or $R^3$ is a phenyl group or a substituted phenyl group.

Still another preferred compounds include those of the general formula (I) in which one of $R^2$ and $R^3$ is an alkyl group and one member selected from the rest of $R^2$ and $R^3$ and $R^1$ is a phenyl group.

Further preferred compounds include those of the general formula (I) in which $R^1$ is a phenyl group which is substituted with one, two or tree substituents, which may be the same or different when present in plural numbers, selected from a lower alkoxy group and a halogen atom, $R^2$ is an alkyl group, a cycloalkyl group, a phenyl group which may be substituted with a halogen atom or a pyridyl group which is substituted with a lower alkoxy group, and $R^3$ is an alkyl group, a cycloalkyl group, a phenyl group or a thienyl group.

Particular examples of the compounds of this invention represented by the general formula (I) will next be shown in Table 1 - Table 2 below.

In Table 2, $R^1$ of the formula (I) is as described below.

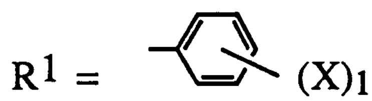

Compound numbers given in these Tables will be referred to in the subsequent descriptions.

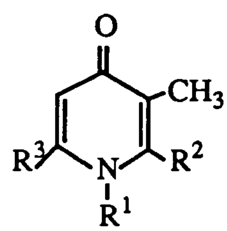

## Table 1

| Compound No. | $R^1$ | $R^2$ | $R^3$ | Melting point (°C) |
|---|---|---|---|---|
| 1 | $-C_3H_7$ | 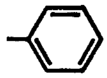 | 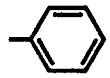 | 148-149 |
| 2 | $-C_4H_9$ | " | " | 137-138 |
| 3 | $-C_4H_9$-i | " | " | 186-187 |
| 4 | $-C_5H_{11}$ | " | " | 116-117 |
| 5 | $-C_5H_{11}$-neo | " | " | 42- 44 |
| 6 | 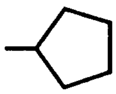 | " | " | 236-239 |
| 7 | 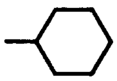 | " | " | 202-204 |
| 8 | 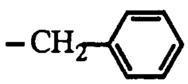 | " | " | 186-187 |
| 9 | 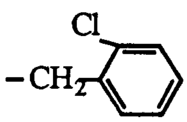 | " | " | 160-163 |
| 10 | 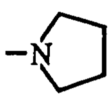 | " | " | 165-167 |

| | | | | |
|---|---|---|---|---|
| 11 | 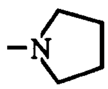 | 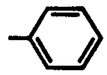 | 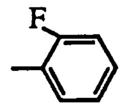 | 146-157 |
| 12 | „ | „ | 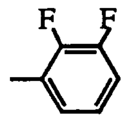 | 118-122 |
| 13 | 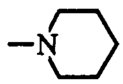 | „ | 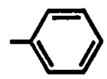 | 200-201 |
| 14 | „ | „ | 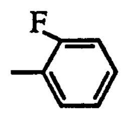 | 198-206 |
| 15 | 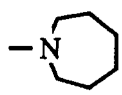 | „ | 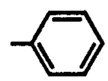 | 202-203 |
| 16 | „ | „ | 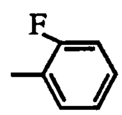 | 177-181 |
| 17 | 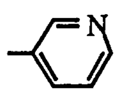 | „ | 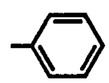 | 215-218 |
| 18 | 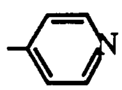 | „ | „ | 196-198 |
| 19 | 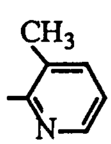 | „ | „ | 224-225 |
| 20 | 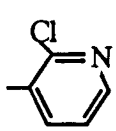 | „ | „ | 268-270 |

| | | | | |
|---|---|---|---|---|
| 21 | 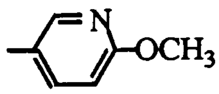 | 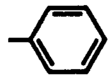 | 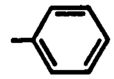 | 258-259 |
| 22 | 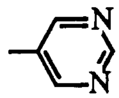 | „ | „ | 193-194 |
| 23 | 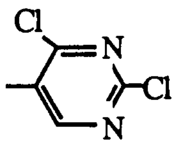 | „ | „ | 218< |
| 24 | 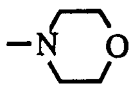 | „ | „ | 195-197 |
| 25 | „ | „ | 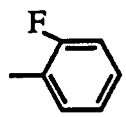 | 186-191 |
| 26 | 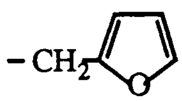 | „ | 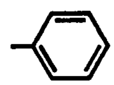 | 136-137 |

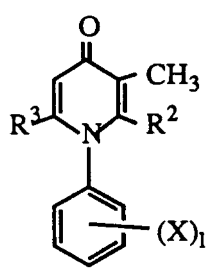

Table 2

| Compound No. | $(X)_1$ | $R^2$ | $R^3$ | Melting point (°C) |
|---|---|---|---|---|
| 27 | H | H | 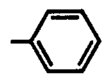 | 222-225 |
| 28 | 2-Cl | " | " | 137-138 |
| 29 | 2-Cl, 5-$OCH_3$ | " | " | 219-221 |
| 30 | 3,5-$(OCH_3)_2$ | " | 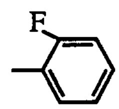 | 165-170 |
| 31 | 3-$CF_3$ | " | 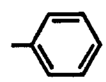 | 196-197 |
| 32 | H | -$CH_3$ | " | 225-226 |
| 33 | 2-Cl | " | " | 201-211 |
| 34 | H | -$C_2H_5$ | " | 169-171 |
| 35 | 2-Cl | " | " | 191-192 |
| 36 | H | -$C_3H_7$-n | " | 176-177 |
| 37 | 2-Cl | " | " | 166-167 |

| | | | | |
|---|---|---|---|---|
| 38 | 3,5-(OCH3)2 | -C3H7-n | 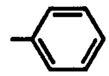 | 159-160 |
| 39 | H | -C3H7-i | „ | 237-239 |
| 40 | 2-Cl | „ | „ | 198-199 |
| 41 | 3,5-(OCH3)2 | „ | „ | 197-199 |
| 42 | 2-Cl, 5-OCH3 | „ | „ | 193-195 |
| 43 | H | 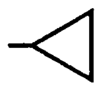 | „ | 209-210 |
| 44 | 2-Cl | „ | „ | 215-216.5 |
| 45 | 3,5-(OCH3)2 | „ | „ | 160-161 |
| 46 | H | -C4H9-n | „ | 123-124 |
| 47 | 2-Cl | „ | „ | 136-137 |
| 48 | 2-Cl, 5-OCH3 | „ | „ | 109-110 |
| 49 | 3,5-(OCH3)2 | „ | „ | 146-147 |
| 50 | H | -C4H9-s | „ | 166-167 |
| 51 | 2-Cl | „ | „ | 134-139 |
| 52 | 3-OCH3 | „ | „ | 186-189 |
| 53 | 3,5-(OCH3)2 | „ | „ | 227-228 |
| 54 | 2-Cl, 5-OCH3 | „ | „ | 134-135 |
| 55 | H | -C4H9-i | -C4H9-i | 109-110 |
| 56 | 2-Cl | „ | „ | 156-157 |
| 57 | 3-OCH3 | „ | „ | 94- 95 |
| 58 | 3,5-(OCH3)2 | „ | „ | 173-174 |
| 59 | 2-Cl, 5-OCH3 | „ | „ | 130-131 |
| 60 | 3,5-(OCH3)2 | „ | -C4H9-s | 166-167 |
| 61 | 2-Cl, 5-OCH3 | „ | „ | 117-121 |

| | | | | |
|---|---|---|---|---|
| 62 | 2-Cl | $-C_4H_9$-i | 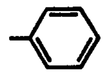 | 193-194 |
| 63 | $3,5-(OCH_3)_2$ | ,, | ,, | 173-174 |
| 64 | 2-Cl, $5-OCH_3$ | ,, | ,, | 182-184 |
| 65 | $3-OCH_3$ | ,, | ,, | 122-124 |
| 66 | H | $-C_5H_{11}$ | ,, | 113-114 |
| 67 | 2-Cl | ,, | ,, | 128-129 |
| 68 | 2-Cl, $5-OCH_3$ | ,, | ,, | 102-103 |
| 69 | $3,5-(OCH_3)_2$ | ,, | ,, | 139-140 |
| 70 | H | 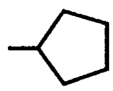 | 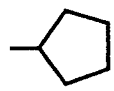 | 185-186 |
| 71 | ,, | ,, | 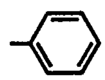 | 176-178 |
| 72 | 2-Cl | ,, | ,, | 197-199 |
| 73 | $3,5-(OCH_3)_2$ | ,, | ,, | 225-226 |
| 74 | $3-OCH_3$ | ,, | ,, | 186-187 |
| 75 | 2-Cl, $5-OCH_3$ | ,, | ,, | 126-128 |
| 76 | H | 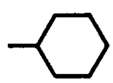 | $-C_4H_9$-i | 156-157 |
| 77 | 2-Cl | ,, | ,, | 225-227 |
| 78 | $3,5-(OCH_3)_2$ | ,, | ,, | 243-244 |
| 79 | 2-Cl, $5-OCH_3$ | ,, | ,, | 175-176 |
| 80 | H | ,, | 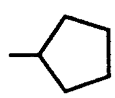 | 170-171 |
| 81 | 2-Cl, $3,5-(OCH_3)_2$ | ,, | ,, | 230-231 |

| | | | | |
|---|---|---|---|---|
| 82 | 2-Cl, 5-$OCH_3$ | 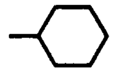 | 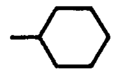 | 239-242 |
| 83 | H | „ | 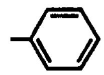 | 228-229 |
| 84 | 2-Cl | „ | „ | 211-212 |
| 85 | 2-Cl, 5-$OCH_3$ | „ | „ | 172-173 |
| 86 | 3-$OCH_3$ | „ | „ | 204-205 |
| 87 | 3,5-$(OCH_3)_2$ | „ | „ | 233-235 |
| 88 | 2-Cl, 3,5-$(OCH_3)_2$ | „ | „ | 247-248 |
| 89 | H | „ | 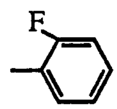 | 229-232 |
| 90 | 2-Cl, 5-$OCH_3$ | „ | „ | 170-171 |
| 91 | H | 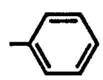 | H | 262-264 |
| 92 | 2-Cl | „ | „ | 190-193 |
| 93 | 3,5-$(OCH_3)_2$ | „ | „ | 206-207 |
| 94 | H | „ | $-C_2H_5$ | 225-226 |
| 95 | 2-Cl | „ | „ | 214-214.5 |
| 96 | 3,5-$(OCH_3)_2$ | „ | „ | 202-203 |
| 97 | H | „ | $-C_3H_7$-n | 194-195 |
| 98 | 2-Cl | „ | „ | 206-207 |
| 99 | 3-Cl | „ | „ | 180-181 |
| 100 | 4-Cl | „ | „ | 264-265 |
| 101 | 2-$CH_3$ | „ | „ | 193-194 |
| 102 | 3-$CH_3$ | „ | „ | 175-176 |
| 103 | 4-$CH_3$ | „ | „ | 237-238 |

| | | | | |
|---|---|---|---|---|
| 104 | 2-$OCH_3$ | 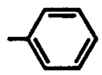 | -$C_3H_7$-n | 173-175 |
| 105 | 2-Br | „ | „ | 198-200 |
| 106 | 2,3-$Cl_2$ | „ | „ | 210-211 |
| 107 | 2-$NO_2$ | „ | „ | 199-201 |
| 108 | 3-$CF_3$ | „ | „ | 174-175 |
| 109 | 3,5-$(OCH_3)_2$ | „ | „ | 211-212 |
| 110 | 3-$OCH_3$ | „ | „ | 180-181 |
| 111 | 2-Cl, 5-$OCH_3$ | „ | „ | 194-197 |
| 112 | 4-$OCH_3$ | „ | „ | 196-197 |
| 113 | H | „ | -$C_3H_7$-i | 203-204 |
| 114 | 2-Cl | „ | „ | 205-206 |
| 115 | 3,5-$(OCH_3)_2$ | „ | „ | 240-241 |
| 116 | 3-$OCH_3$ | „ | „ | 206-207 |
| 117 | 2-Cl, 5-$OCH_3$ | „ | „ | 203-204 |
| 118 | H | „ | 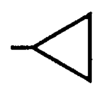 | 212-213.5 |
| 119 | 2-Cl | „ | „ | 200-203 |
| 120 | 3-$OCH_3$ | „ | „ | 194-195 |
| 121 | 3,5-$(OCH_3)_2$ | „ | „ | 212-213 |
| 122 | H | „ | -$C_4H_9$-n | 161-162 |
| 123 | 2-Cl | „ | „ | 155-156 |
| 124 | 3,5-$(OCH_3)_2$ | „ | „ | 187-188 |
| 125 | 3-$OCH_3$ | „ | „ | 146-148 |
| 126 | 2-Cl, 5-$OCH_3$ | „ | „ | 165-166 |
| 127 | H | „ | -$C_4H_9$-s | 158-159 |

| | | | | |
|---|---|---|---|---|
| 128 | 2-Cl | 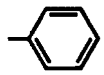 | $-C_4H_9$-s | 172-175 |
| 129 | $3-OCH_3$ | „ | „ | 183-185 |
| 130 | $3,5-(OCH_3)_2$ | „ | „ | 206-207 |
| 131 | 2-Cl, $3,5-(OCH_3)_2$ | „ | „ | 220-221 |
| 132 | 2-Cl, $5-OCH_3$ | „ | „ | 159-161 |
| 133 | H | „ | $-C_4H_9$-i | 166-168 |
| 134 | 2-Cl | „ | „ | 173-174 |
| 135 | $3-OCH_3$ | „ | „ | 174-175 |
| 136 | $3,5-(OCH_3)_2$ | „ | „ | 237-238 |
| 137 | 2-Cl, $5-OCH_3$ | „ | „ | 192-194 |
| 138 | H | „ | $-C_4H_9$-t | 205-208 |
| 139 | 2-Cl | „ | „ | 198-200 |
| 140 | $3,5-(OCH_3)_2$ | „ | „ | 260-264 |
| 141 | H | „ | $-C_5H_{11}$-n | 135-136 |
| 142 | 2-Cl | „ | „ | 138-139 |
| 143 | $3-OCH_3$ | „ | „ | 126-127 |
| 144 | $3,5-(OCH_3)_2$ | „ | „ | 150-151 |
| 145 | 2-Cl, $5-OCH_3$ | „ | „ | 151-152 |
| 146 | H | „ | 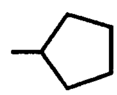 | 229-230 |
| 147 | 2-Cl | „ | „ | 242-243 |
| 148 | 3-Cl | „ | „ | 227-228 |
| 149 | 4-Cl | „ | „ | 268-269 |
| 150 | $2-CH_3$ | „ | „ | 212-213 |
| 151 | $3-CH_3$ | „ | „ | 198-200 |
| 152 | $4-CH_3$ | „ | „ | 192-193 |

| | | | | |
|---|---|---|---|---|
| 153 | 2-OCH3 | 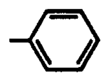 | 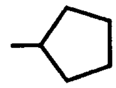 | 216-217 |
| 154 | 3-OCH3 | ,, | ,, | 187-189 |
| 155 | 4-OCH3 | ,, | ,, | 212-213 |
| 156 | 3,5-(OCH3)2 | ,, | ,, | 247-248 |
| 157 | 2-Cl, 5-OCH3 | ,, | ,, | 211-212 |
| 158 | 2,3-Cl2 | ,, | ,, | 227-228 |
| 159 | 2-NO2 | ,, | ,, | 238-239 |
| 160 | 3-CF3 | ,, | ,, | 181-182 |
| 161 | 2-Cl, 3,5-(OCH3)2 | ,, | ,, | 219-220 |
| 162 | H | ,, | 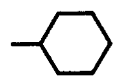 | 195-203 |
| 163 | 2-Cl | ,, | ,, | 241-242 |
| 164 | 3-OCH3 | ,, | ,, | 213.5-214 |
| 165 | 3,5-(OCH3)2 | ,, | ,, | 237-238 |
| 166 | 2-Cl, 5-OCH3 | ,, | ,, | 193-194 |
| 167 | 2-Cl, 3,5-(OCH3)2 | ,, | ,, | 254-255 |
| 168 | 3-CF3 | ,, | ,, | 226-227 |
| 169 | 2-Cl, 5-OCH3 | ,, | 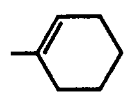 | 173-175 |
| 170 | H | ,, | 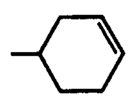 | 228-230 |
| 171 | 2-Cl, 5-OCH3 | ,, | ,, | 208-210 |
| 172 | H | ,, | -C7H15-n | 109-110 |
| 173 | 2-Cl | ,, | ,, | 107-108 |
| 174 | 3-OCH3 | ,, | ,, | 104-106 |

| | | | | |
|---|---|---|---|---|
| 175 | 3,5-($OCH_3$)2 | 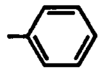 | -$C_7H_{15}$-n | 110-111 |
| 176 | 2-Cl, 5-$OCH_3$ | " | " | 118-120 |
| 177 | H | " | 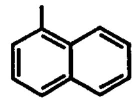 | 192-194 |
| 178 | " | " | 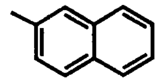 | 219-220 |
| 179 | " | " | 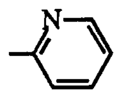 | 201-202 |
| 180 | " | " | 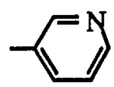 | 224-226 |
| 181 | " | " | 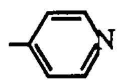 | 219-221 |
| 182 | " | " | 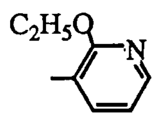 | 215-217 |
| 183 | " | " | 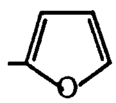 | 224-225 |
| 184 | 2-Cl | " | " | 218-220 |
| 185 | H | " | 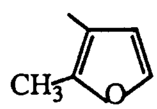 | 210-212 |
| 186 | 2-Cl | " | " | 218-219 |

| | | | | |
|---|---|---|---|---|
| 187 | 3,5-(OCH3)2 | 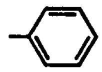 | 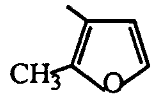 | 265.5-267 |
| 188 | 2-Cl, 5-OCH3 | " | " | 255-256 |
| 189 | H | " | 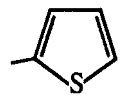 | 251-252 |
| 190 | 2-Cl | " | " | 233-234 |
| 191 | 3,5-(OCH3)2 | " | " | 249-250 |
| 192 | H | 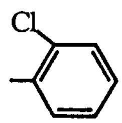 | -C4H9-s | 169-178 |
| 193 | 2-Cl | " | " | 163-165 |
| 194 | 2-Cl | " | " | 171-173 |
| 195 | 3-OCH3 | " | " | 176-180 |
| 196 | 3,5-(OCH3)2 | " | " | 185-189 |
| 197 | 2-Cl, 5-OCH3 | " | " | 178-181 |
| 198 | 2-Cl, 5-OCH3 | " | " | 185-186 |
| 199 | H | " | -C4H9-i | 215-217 |
| 200 | 2-Cl | " | " | 186-188 |
| 201 | 2-Cl | " | " | 149-151 |
| 202 | 3-OCH3 | " | " | 186-188 |
| 203 | 3,5-(OCH3)2 | " | " | 205-206 |
| 204 | 2-Cl, 5-OCH3 | " | " | 186-188 |
| 205 | 2-Cl, 5-OCH3 | " | " | 236-240 |
| 206 | H | " | 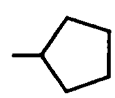 | 221-223 |

| | | | | |
|---|---|---|---|---|
| 207 | 2-Cl | 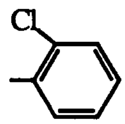 | 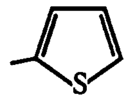 | 194-195 |
| 208 | 3,5-$(OCH_3)_2$ | ,, | ,, | 229-231 |
| 209 | 3,5-$(OCH_3)_2$ | 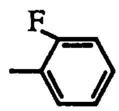 | H | 215-217 |
| 210 | 2-Cl | ,, | 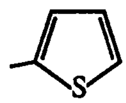 | 218-219 |
| 211 | 2-Cl | ,, | ,, | 210-211 |
| 212 | 2-Cl, 5-$OCH_3$ | ,, | ,, | 271-273 |
| 213 | 2-Cl, 5-$OCH_3$ | ,, | ,, | 264-265 |
| 214 | 2-Cl, 3,5-$(OCH_3)_2$ | ,, | ,, | 242-245 |
| 215 | ,, | ,, | ,, | 230-235 |
| 216 | 3-$OCH_3$ | ,, | ,, | 234-235 |
| 217 | 3,5-$(OCH_3)_2$ | ,, | ,, | 214-215 |
| 218 | H | 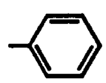 | 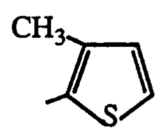 | 205-207 |
| 219 | ,, | ,, | 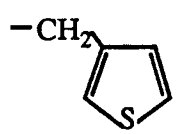 | 189-190 |
| 220 | ,, | 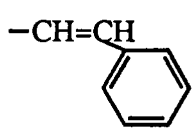 | 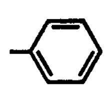 | 142-144 |
| 221 | 2-Cl | ,, | ,, | 155-156 |

| | | | | |
|---|---|---|---|---|
| 222 | 3,5-(OCH3)2 | 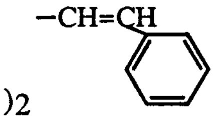 | 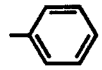 | 185-187 |
| 223 | H | 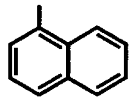 | „ | 252-254 |
| 224 | „ | 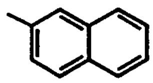 | „ | 129-130 |
| 225 | „ | 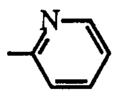 | „ | 214-215 |
| 226 | „ | 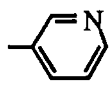 | „ | 216-219 |
| 227 | „ | 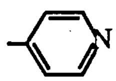 | „ | 218-223 |
| 228 | 2-Cl | 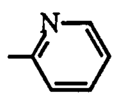 | „ | 193-194 |
| 229 | 3,5-(OCH3)2 | „ | „ | 220-221 |
| 230 | „ | 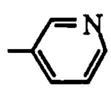 | „ | 232-235 |
| 231 | 2-Cl, 5-OCH3 | 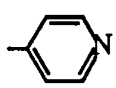 | „ | 208-210 |
| 232 | 3,5-(OCH3)2 | „ | „ | 223-225 |
| 233 | H | 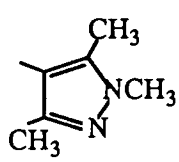 | „ | 222-224 |

| | | | | |
|---|---|---|---|---|
| 234 | 2-Cl | 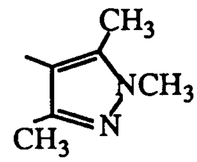 | 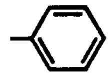 | 206-208 |
| 235 | 3,5-$(OCH_3)_2$ | " | " | 205-207 |
| 236 | H | 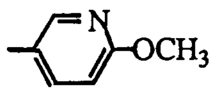 | " | 233-234 |
| 237 | 3,5-$(OCH_3)_2$ | " | " | 201-203 |
| 238 | H | 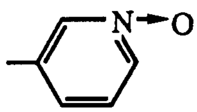 | " | 268.5-270.5 |
| 239 | " | 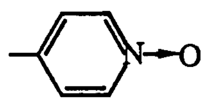 | " | 232-234 |
| 240 | 3,5-$(OCH_3)_2$ | " | " | 192-195 |
| 241 | 2-Cl, 5-$OCH_3$ | -$CH_2OCH_3$ | " | 147-150 |
| 242 | 2-Cl | -$CH_2OC_2H_5$ | " | 92-95 |
| 243 | H | 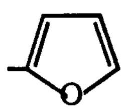 | " | 182-184 |
| 244 | 2-Cl | " | " | 186-192 |
| 245 | 3,5-$(OCH_3)_2$ | " | " | 178-184 |
| 246 | H | 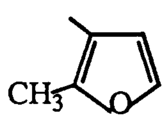 | " | 192-194 |
| 247 | " | 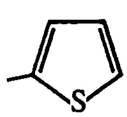 | " | 227-229 |

| | | | | |
|---|---|---|---|---|
| 248 | H | 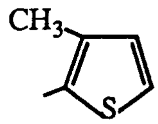 | 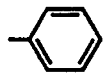 | 202-204 |

In Table 2, pairs of Compound 193 and Compound 194; Compound 197 and Compound 198; Compound 200 and Compound 201; Compound 204 and Compound 205; Compound 210 and Compound 211; Compound 212 and Compound 213; Compound 214 and Compound 215 are respectively in the relationship of atropisomers.

Preferred examples of the compounds of the general formula (I) include Compounds 42, 49, 54, 85, 88, 117, 131, 132, 157, 161, 165, 166, 190, 193, 196, 200, 217 and 237. Of these, Compounds 54, 85, 88, 131, 132, 161, 165, 166 and 217 are particularly preferred.

Descriptions will next be made of the production of the compounds of formula (I) according to this invention.

The compounds of this invention may each be produced by reacting a 1,5-disubstituted pentanetrione derivative represented by the general formula (II):

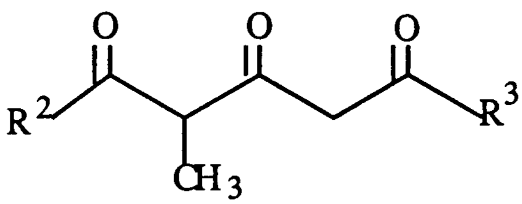

(II)

wherein $R^2$ and $R^3$ have the same meanings as defined above, or a tautomer thereof, with an amine derivative represented by the general formula (III):

$R^1$-$NH_2$ (III)

wherein $R^1$ has the same meaning as defined above.

The tautomers of the above compound (II) are represented by the following formula:

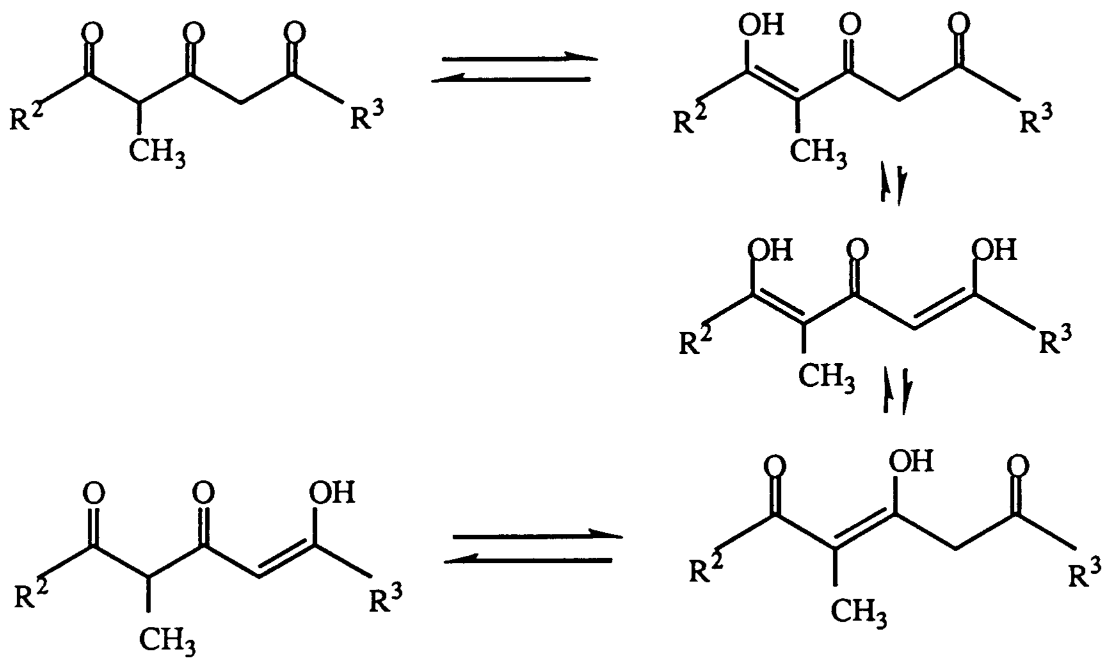

Process (1)

In the process, the reaction between the pentanetrione of the formula (II) or its tautomer and the amine derivative of the formula (III) may be conducted generally by dissolving or suspending both the reactants in a suitable solvent, for example, a hydrocarbon such as benzene, toluene or xylene, a halogenated hydrocarbon such as chlorobenzene, dichlorobenzene or chloroform, an ether such as dimethoxyethane, tetrahydrofuran or dioxane, an amide such as N,N-dimethylformamide, N,N-dimethylacetamide or N-methylpyrrolidone, dimethylsulfoxide, acetonitrile, acetic acid, or the like. It is however preferable to carry out the reaction by dissolving the reactants in xylene or chlorobenzene.

Here, the reaction may be allowed to proceed with or without addition of one or more suitable reaction aids. As usable reaction aids, may be mentioned suitable acids, for example, mineral acids such as sulfuric acid and hydrochloric acid, organic acids such as p-toluenesulfonic acid and acetic acid, and Lewis acids such as boron trifluoride, aluminum chloride and titanium tetrachloride, and polyphosphoric acid. In addition, the reaction may also be allowed to proceed while collecting the resultant water in a Dean-Stark trap.

Depending on the kinds of the acid and solvent to be employed, the reaction may also be conducted in the presence of a dehydrating agent such as molecular sieve. When p-toluenesulfonic acid is used in dimethylsulfoxide, for instance, Molecular Sieves 5A may be used. The molecular sieve can be used generally in an amount of 2-200 g, preferably, 50-150 g per 0.1 mole of the 1,5-disubstituted pentanetrione derivative (II). The reaction may be carried out at a desired temperature between the solidifying point of the solvent and its boiling point, preferably, at a temperature in a range of from 10 °C to the boiling point of the solvent.

After completion of the reaction, the acid or molecular sieve is removed respectively by washing with water and an alkali solution or by filtration or the like. Upon removal of the solvent by distillation subsequent to extraction of the reaction product with chloroform, the compound of this invention can be obtained. The compound of this invention may be purified by recrystallizing same from acetone, methanol, ethanol, benzene, toluene, diisopropyl ether, ethyl acetate, chloroform, hexane or the like, or subjecting same to chromatography on a silica gel column, if necessary.

Process (2)

The compound of this invention can also be produced by reacting a substituted propiolate derivative represented by the general formula (IV):

$R^3$-C≡CCOOC$_2$H$_5$ (IV)

wherein $R^3$ has the same meanings as defined above, with a ketimine derivative represented by the general formula (V):

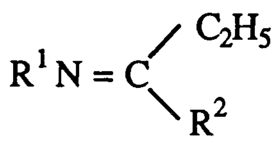

(V)

wherein $R^1$ and $R^2$ have the same meanings as defined above, in the presence of 0.5-5 equivalents of a Lewis acid such as aluminum chloride, titanium tetrachloride, trifluoroborate, trichloroborate or the like as a reaction aid.

Upon conducting the reaction in accordance with the process (2), the substituted propiolate derivative of the formula (IV) and the ketimine derivative of the formula (V) are dissolved or suspended in a suitable solvent, for example, benzene, toluene, xylene, chlorobenzene, dimethylsulfoxide or the like. As an alternative, the reaction may also be carried out without any solvent. It is however preferable to dissolve both the reactants in toluene and then to add 1 equivalent of aluminum chloride.

After completion of the reaction, the reaction mixture is washed successively with an aqueous solution of hydrochloric acid, water and an alkali solution. After extraction with chloroform, the resultant chloroform solution is dried and the solvent is then distilled off to obtain the compound of this invention. If necessary, the compound of this invention can be purified by subjecting it to chromatography on a silica gel column for its crystallization and then recrystallizing same from acetone, methanol, benzene, ethyl acetate, chloroform, n-hexane or the like.

Production of the compounds of this invention will now be illustrated with reference to the following Examples.

Example 1

Synthesis of 1-cyclohexyl-3-methyl-2,6-diphenyl-4(1H)-pyridinone (Compound 7)

To 150 ml of xylene were added 2.8 g of 2-methyl-1,5-diphenyl-1,3,5-pentanetrione, 9.8 g of cyclohexylamine and 1 ml of titanium tetrachloride, followed by refluxing the reaction mixture for 2 hours. After cooling the reaction mixture, the reaction mixture was poured into 1 liter of water and extracted with chloroform. The organic layer was washed first with 300 ml of 10% hydrochloric acid and then with 300 ml of 10% aqueous solution of sodium hydroxide. The organic layer was washed further with water and was then dried over anhydrous sodium sulfate. Subsequent to removal of the sodium sulfate by filtration, the solvent was distilled off and the residue was subjected to chromatography on a silica gel column (eluent: chloroform) to afford crystals. Upon recrystallization of the resultant crystals from a mixed solvent of ethyl acetate and hexane, 0.4 g of 1-cyclohexyl-3-methyl-2,6-diphenyl-4(1H)-pyridinone having a melting point of 202-204 °C was obtained.

Example 2;

Synthesis of 3-methyl-1-morpholino-2,6-diphenyl-4(1H)-pyridinone (Compound 24)

To 150 ml of toluene were added 4.4 g of N-morpholino-1-phenylpropanimine, 3.5 g of ethyl phenylpropiolate and 3.2 g of aluminum chloride. The reaction mixture was then heated under reflux for 10 hours. After cooling, the reaction mixture was poured into 300 ml of 2N sulfuric acid which had been ice-cooled, followed by extraction with dichloromethane. After washing the organic layer with 150 ml of 10% sodium hydroxide and then water, it was dried over anhydrous sodium sulfate. Subsequent to removal of the sodium sulfate, the solvent was distilled off and the residue was subjected to column chromatography on silica gel (eluent: chloroform) to afford crystals. The resulting crystals were recrystallized from a mixed solvent of ethyl acetate and hexane, thereby affording 0.7 g of 3-methyl-1-morpholino-2,6-diphenyl-4(1H)-pyridinone having a melting point of 195-197 °C.

Example 3

Synthesis of 3-methyl-2,6-diphenyl-1-(pyrrolidin-1-yl)-4(1H)-pyridinone (Compound 10)

To 150 ml of xylene were added 3.6 g of 1-aminopyrrolidine hydrochloride, 2.9 g of triethylamine. The reaction mixture was refluxed for 10 minutes. After cooling, to the mixture was added 6.0 g of 2-methyl-1,5-diphenyl-1,3,5-pentanetrione and 3.6 g of p-toluenesulfonic acid hydrate and then the reaction mixture was refluxed for 5 hours. The xylene of the resultant mixture was distilled off and the residue was extracted with 150 ml of chloroform. The organic layer was washed first with 100 ml of 10% hydrochloric acid and then with 100 ml of a 10% aqueous solution of sodium hydroxide. After washing the solution in chloroform further with water, it was dried over anhydrous sodium sulfate. Subsequent to removal of the sodium sulfate, the solvent was distilled off and the residue was subjected to column chromatography on silica gel (eluent: ethyl acetate) to obtain 0.2 g of 3-methyl-2,6-diphenyl-1-(pyrrolidin-1-yl)-4(1H)-pyridinone having a melting point of 165-167 °C.

Example 4

Synthesis of 1-isobutyl-3-methyl-2,6-diphenyl-4(1H)-pyridinone (Compound 3)

In 100 ml of chlorobenzene were dissolved 4.0 g of 2-methyl-1,5-diphenyl-1,3,5-pentanetrione and 6.2 g of isobutylamine and to the solution was added 1.9 g of titanium tetrachloride. The reaction mixture was refluxed for 2 hours. After cooling, the chlorobenzene of the reaction mixture was distilled off and the residue was extracted with 100 ml of chloroform. The organic layer was washed first with 50 ml of 10% hydrochloric acid and then with 50 ml of a 10% aqueous solution of sodium hydroxide. After washing the solution in chloroform further with water, it was dried over anhydrous sodium sulfate. Subsequent to removal of the sodium sulfate, the solvent was distilled off and the residue was subjected to column chromatography on silica gel (eluent: ethyl acetate). The resulting crystals were recrystallized from a 1/5 mixed solvent of ethyl acetate and hexane, thereby affording 0.7 g of 1-isobutyl-3-methyl-2,6-diphenyl-4(1H)-pyridinone having a melting point of 186-187 °C.

Example 5

Synthesis of 4-[3-methyl-1,6-diphenyl-4(1H)-pyridinon-2-yl]pyridine N-oxide (Compound 239)

To a solution of 0.9 g of 3-methyl-1,6-diphenyl-2-(4-pyridyl)-4(1H)-pyridinone in chloroform was added 0.7 g of m-chloroperbenzoic acid and the reaction mixture was stirred for 4 hours. To the reaction mixture were added water and a saturated aqueous solution of sodium hydrogencarbonate, followed by extraction with chloroform. The organic layer was washed with water and it was dried over anhydrous magnesium sulfate. Subsequent to removal of the magnesium sulfate, the solvent was distilled. The resultant crystals were recrystallized from ethyl acetate, yielding 0.8 g of 4-[3-methyl-1,6-diphenyl-4(1H)-pyridinone-2-yl]-pyridine N-oxide having a melting point of 232-234 °C.

Example 6

Synthesis of 1-(2-chlorophenyl)-3-methyl-6-(2-methylfuran-3-yl)-2-phenyl-4(1H)-pyridinone (Compound 186)

In 500 ml of xylene were dissolved 5.5 g of 2-methyl-5-(2-methylfuran-3-yl)-1-phenyl-1,3,5-pentanetrione, 3.8 g of 2-chloroaniline and 5.7 g of p-toluenesulfonic acid. The reaction mixture was refluxed for 5 hours. After cooling the reaction mixture, the xylene was distilled off and the residue was extracted with chloroform. The organic layer was washed first with 500 ml of 10% hydrochloric acid and then with 300 ml of a aqueous solution of sodium hydroxide. After washing the chloroform phase further with water, the solution in chloroform was dried over anhydrous sodium sulfate. Subsequent to removal of the sodium sulfate, the solvent was distilled off and the residue was purified by column chromatography on silica gel (eluent: ethyl acetate - n-hexane) to afford crude crystals. The crystals were recrystallized from acetone, yielding 1.1 g of 1-(2-chlorophenyl)-3-methyl-6-(2-methylfuran-3-yl)-2-phenyl-4(1H)-pyridinone having a melting point of 218-219 °C.

Example 7

Synthesis of 3-methyl-1,2-diphenyl-6-(pyridin-3-yl)-4(1H)-pyridinone (Compound 180)

To 700 ml of xylene were added 62.8 g of N-phenyl-1-phenylpropanimine, 32.2 g of ethyl (pyridin-3-yl)-propiolate and 40 g of aluminum chloride, followed by heating and reaction mixture with stirring at 60 °C for 3 days. After cooling, the reaction mixture was poured into 2-N sulfuric acid, followed by extraction with chloroform. After washing the organic layer with 400 ml of a 10% aqueous solution of sodium hydroxide and then successively with water. The filtrate was then dried over anhydrous sodium sulfate, the solvent was distilled off and the residue was purified by column chromatography on silica gel (eluent: ethyl acetate - n-hexane) to afford crude crystals. The crystals were recrystallized from a mixed solvent of acetone and n-hexane to give 2.3 g of 3-methyl-1,2-diphenyl-6-(pyridin-3-yl)-4(1H)-pyridinone having a melting point of 224-226 °C.

Example 8

Synthesis of 1-(2-chloro-5-methoxyphenyl)-6-isopropyl-3-methyl-2-phenyl-4(1H)-pyridinone (Compound 117)

To 200 ml of xylene were added 4.9 g of 2,6-dimethyl-1-phenyl-1,3,5-heptanetrione, 6.6 g of 2-chloro-5-methoxyaniline hydrochloride, 4.4 g of p-toluenesulfonic acid and 15.9 g of Molecular Sieves 5A, followed by refluxing the reaction mixture for 2 hours. After cooling the reaction mixture, solid matter was removed from the reaction mixture, followed by addition of 200 ml of chloroform thereto. The organic layer was washed first with 500 ml of 10% hydrochloric acid and then with 500 ml of a 10% aqueous solution of sodium sulfate. After washing the organic layer further with water, it was dried over anhydrous sodium sulfate. Subsequent to removal of sodium sulfate, the solvent was distilled off and the residue was subjected to column chromatography on silica gel (eluent: ethyl acetate). The resultant crystals were recrystallized from ethyl acetate, thereby yielding 1.4 g of 1-(2-chloro-5-methoxyphenyl)-6-isopropyl-3-methyl-2-phenyl-4(1H)-pyridinone having a melting point of 203-204 °C.

Example 9

Synthesis of 1-(2-chlorophenyl)-3-methyl-2-phenyl-4(1H)-pyridinone (Compound 92)

To 200 ml of toluene were added 6.1 g of N-(2-chlorophenyl)-1-phenylpropanimine, 2.5 g of ethyl propiolate and 4.1 g of aluminum chloride, followed by stirring at room temperature for 1 hour. The reaction mixture was washed first with 100 ml of 2N sulfuric acid and then with water, it was dried over anhydrous sodium sulfate. Subsequent to removal of the sodium sulfate, to the reaction mixture were added 200 ml of toluene and 3.7 g of aluminum chloride, followed by refluxing for 2 hours. After allowing the reaction mixture to cool down, it was poured into 100 ml of 2N sulfuric acid, followed by extraction of 500 ml of chloroform. The organic layer was washed with a 10% aqueous solution of sodium hydroxide and then with water, it was dried over anhydrous sodium sulfate. After removal of the sodium sulfate, the solvent was distilled off and the residue was subjected to column chromatography on silica gel (eluent: chloroform / methanol =100/1) . Upon recrystallization of the resultant crystals, from a 2:1 mixed solvent of n-hexane and acetone, 0.2 g of 1-(2-chlorophenyl)-3-methyl-2-phenyl-4(1H)-pyridinone having a melting point of 190-193 °C.

Example 10

Synthesis of 1-(2-chloro-3,5-dimethoxyphenyl)-6-sec-butyl-3-methyl-2-phenyl-4(1H)-pyridinone (Compound 131)

To 500 ml of xylene were added 5.2 g of 2,6-dimethyl-1-phenyl-1,3,5-octanetrione, 5.2 g of 2-chloro-3,5-dimethoxyaniline and 5.7 g of p-toluenesulfonic acid, followed by reflux the reaction mixture with a Dean-Stark trap for 1 hour. After cooling, the reaction mixture was poured into water, followed by addition of 200 ml of chloroform. After extraction with chloroform, the organic layer was washed first with a 10% aqueous solution of sodium sulfate and then with 10% hydrochloric acid. After washing the solution in chloroform further with water, it was dried over anhydrous sodium sulfate. Subsequent to removal of the sodium sulfate, the solvent was distilled off and the residue was purified by column chromatography on silica gel (eluent:

1:1 mixed solvent of n-hexane and chloroform). The resulting crystals were recrystallized from acetone, thereby affording 2.3 g of 1-(2-chloro-3,5-dimethoxyphenyl)-6-sec-butyl-3-methyl-2-phenyl-4(1H)-pyridinone having a melting point of 220-221 °C.

Example 11

Synthesis of 6-sec-butyl-2-(2-chlorophenyl)-1-(3,5-dimethoxyphenyl)-3-methyl-4(1H)-pyridinone (Compound 196)

To 300 ml of xylene were added 5.3 g of 1-(2-chlorophenyl)-2,6-dimethyl-1,3,5-octanetrione, 6.3 g of 3,5-dimethoxyaniline, 4.3 g of p-toluenesulfonic acid and 10 g of Molecular Sieves 5A, followed by refluxing the reacting mixture with a Dean-Stark trap for 3 hour. After cooling the reaction mixture, solid matter was removed from the reaction mixture, followed by addition of 200 ml of chloroform thereto. The organic layer was washed first with 100 ml of an aqueous solution of sodium hydroxide and then with 100 ml of 10% hydrochloric acid. The organic layer was washed further with water and was then dried over anhydrous sodium sulfate. Subsequent to removal of the sodium sulfate by filtration, the solvent was distilled off and the residue was purified by column chromatography on silica gel (eluent: ethyl acetate) to afford crude crystals. The crystals were recrystallized from ethyl acetate, 0.37 g of 6-sec-butyl-2-(2-chlorophenyl)-1-(3,5-dimethoxyphenyl)-3-methyl-4(1H)-pyridinone having a melting point of 185-189 °C.

Example 12

Synthesis of 1-(3,5-dimethoxyphenyl)-2-(4-methoxypyridin-3-yl)-3-methyl-6-phenyl-4(1H)-pyridinone (Compound 237)

To 500 ml of chlorobenzene were added 3.2 g of 1-(4-methoxypyridin-3-yl)-2-methyl-5-phenyl-1,3,5-pentanetrione, 3.3 g of 3,5-dimethoxyaniline, 4.0 g of p-toluenesulfonic acid and 10 g of Molecular Sieves 5A, followed by refluxing the reaction mixture for 2 hours. After cooling the reaction mixture, solid matter was removed from the reaction mixture, followed by addition of 500 ml of chloroform thereto. The organic layer was washed first with 1000 ml of 10% hydrochloric acid and then with 1000 ml of a 10% aqueous solution of sodium hydroxide. The organic layer was washed further with water and then dried over anhydrous sodium sulfate. Subsequent to removal of the sodium sulfate by filtration, the solvent was distilled off and the residue was subjected to column chromatography on silica gel (eluent: ethyl acetate). Upon recrystallization of the resultant crystals from a 2:1 mixed solvent of acetone and hexane, 1.5 g of 1-(3,5-dimethoxyphenyl)-2-(4-methoxypyridin-3-yl)-3-methyl-6-phenyl-4(1H)-pyridinone having a melting point of 201-203 °C.

Example 13

Synthesis of 2-(2-chlorophenyl)-1-(3,5-dimethoxyphenyl)-3-methyl-6-(2-thienyl)-4-(1H)-pyridinone (Compound 208)

To 500 ml of xylene were 2.6 g of N-(3,5-dimethoxyphenyl)-1-(2-chlorophenyl)propanimine, 2.5 g of ethyl (2-thienyl) propiolate and 1.4 g of aluminum chloride, followed by refluxing for 15 hours. After cooling, the reaction mixture was poured into 1000 ml of 2N sulfuric acid which had been ice-cooled, followed by extraction with chloroform. After washing the organic layer with water and then with a 10% of aqueous solution of sodium hydroxide, it was then dried over anhydrous sodium sulfate. Subsequent to removal of the sodium sulfate, the solvent was distilled off. The residue was purified by column chromatography on silica gel (eluent: a mixed solvent of ethyl acetate and n-hexane). The resulting crystals were recrystallized from a mixed solvent of n-hexane and acetone, thereby affording 0.3 g of 2-(2-chlorophenyl)-1-(3,5-dimethoxyphenyl)-3-methyl-6-(2-thienyl)-4(1H)-pyridinone having a melting point of 229-231 °C.

Example 14

Synthesis of 1-(2-chloro-3,5-dimethoxyphenyl)-2-cyclohexyl-6-cyclopentyl-3-methyl-4(1H)-pyridinone (Compound 81)

In 150 ml of chlorobenzene were dissolved 4.5 g of 1-cyclohexyl-5-cyclopentyl-2-methyl-1,3,5-pentanetrione and 6.0 g of 2-chloro-3,5-dimethoxyaniline. To the mixture was added 2.0 g of titanium tetrachloride, followed by refluxing for 4 hours. The chlorobenzene of the reaction mixture was distilled off and the residue was extracted with 150 ml of chloroform. The organic layer was washed first with 100 ml of 10% hydrochloric acid and then with 100 ml of a 10% aqueous solution of sodium hydroxide. After washing the organic layer with water, it was dried over anhydrous sodium sulfate and the solvent was distilled off. The residue was purified by column chromatography on silica gel (eluent: chloroform). The resulting crystals were recrystallized from ethyl acetate, affording 0.6 g of 1-(2-chloro-3,5-dimethoxyphenyl)-2-cyclohexyl-6-cyclopentyl-3-methyl-4(1H)-pyridinone having a melting point of 230-231 °C.

Example 15

Synthesis of 1-(2-chloro-5-methoxyphenyl)-2,6-diisobutyl-3-methyl-4(1H)-pyridinone (Compound 59)

To 200 ml of xylene were added 1.8 g of 2,5,10-trimethyl-4,6,8-undecanetrione, 3.1 g of 2-chloro-5-methoxyaniline hydrochloride, 1.6 g of p-toluenesulfonic acid and 5.4 g of Molecular Sieves 5A, followed by refluxing with a Dean-Stark trap for 3 hours. After cooling the reaction mixture, solid matter was filtered off and the filtrate was evaporated. The residue was dissolved in 200 ml of chloroform and the organic layer was washed first with 100 ml of a 10% aqueous solution of sodium hydroxide and then with 10% hydrochloric acid. After washing with water, the organic layer was dried over anhydrous sodium sulfate. Subsequent to removal the sodium sulfate, the solvent was distilled off and the residue was purified by column chromatography on silica gel (eluent: ethyl acetate). Upon recrystallization of the resultant crystals from ethyl acetate, 1.03 g of 1-(2-chloro-5-methoxyphenyl)-2,6-diisobutyl-3-methyl-4(1H)-pyridinone having a melting point of 130-131 °C.

The new compound of the formula (I) according to this invention may be applied alone or in the form of a composition. They may usually be formulated into a fungicidal composition by mixing with a liquid or solid carrier or vehicle which is conventionally used in the art. According to another aspect of this invention, therefore, there is provided a fungicidal composition for agricultural and horticultural utilities, which comprises a compound of the general formula (I) as defined hereinbefore or a salt thereof as the active ingredient, in combination with a carrier for the active ingredient.

Although the new compound of this invention may be applied alone, it may usually be admixed with a carrier, optionally together with surfactant, dispersant or auxiliary agent and then formulated in a known manner, for example, into a dust, a wettable powder, an emulstifiable concentrate, fine particles or granules.

As suitable examples of the carriers, may be mentioned solid carriers such as talc, bentonite, clay, kaolin, diatomaceous earth, white carbon, vermiculite, slaked lime, siliceous sand, ammonium sulfate and urea; and liquid carriers such as isopropyl alcohol, xylene, cyclohexanone, and methylnaphthalene. Illustrative examples of the surfactant and dispersant may include salts of alcohol-sulfuric acid esters, alkylarylsulfonic acid salts, ligninsulfonic acid salts, diarylmethanedisulfonic acid salts, polyoxyethylene glycol ethers, polyoxyethylene alkyl aryl ethers, polyoxyethylenesorbitan monoalkylates, and so on. Suitable examples of the adjuvants include carboxymethylcellulose, polyethylene glycol, gum arabic, etc. These preparations can be applied after diluting same to a suitable concentration of the active ingredient, or they can be applied directly.

The proportion of the active ingredient in the composition may be increased or decreased as needed. When formulated into a dust or granules, 0.1-20 wt.% of the active ingredient is preferred. For an emulsifiable concentrate or wettable powder, 5-80 wt.% of the active ingredient is preferred.

The rate of application of the fungicidally active compound of this invention may vary depending on the type of the active compound employed, the kind of disease to be controlled, the nature of occurrence of the disease, the degree of damage, environmental conditions, the preparation form to be used, etc. When the composition of this invention is applied directly in the form of dust or granules, it is recommendable that the rate of application of the active ingredient is suitably chosen in a range of 0.1-5 Kg per 10 ares, 1 g-1 Kg of the active ingredient per 10 ares is preferred. When the composition of this invention in the form of an emulsifiable concentrate or a wettable powder is diluted with water before its application and then the liquid preparation obtained is applied, it is preferred that the concentration of the active ingredient in the diluted

liquid preparation is suitably chosen in a range of 0.1-10000 ppm, preferably 10-3000 ppm.

The fungicidal composition of this invention are now illustrated with reference to the following Examples 16-19, wherein all designations of "%" are given in percent by weight.

Example 16: (Dust)

Two percent of Compound 6, 5% of diatomaceous earth and 93% of clay were uniformly mixed and ground into a dust.

Example 17: (Wettable powder)

Fifty percent of Compound 7, 45% of diatomaceous earth, 2% of sodium dinaphthylmethanedisulfonate and 3% of sodium ligninsulfonate were uniformly mixed and ground into a wettable powder.

Example 18: (Emulsifiable concentrate)

Thirty percent of Compound 17, 20% of cyclohexanone, 11% of polyoxyethylene alkylaryl ether, 4% of calcium alkylbenzenesulfonate and 35% of methylnaphthalene were evenly dissolved together to prepare an emulsifiable concentrate.

Example 19: (Granules)

Five percent of Compound 18, 2% of the sodium salt of the ester of lauryl alcohol and sulfuric acid, 5% of sodium ligninsulfonate, 2% of carboxymethylcellulose and 86% of clay were mixed together and ground uniformly. The mixture was added and kneaded with 20% of water. The resulting mixture was formed into granules of 14-32 mesh by means of an extrusion granulator and was then dried into granules.

The fungicidal compounds of this invention have excellent properties. They show broad antibacterial and antifungal spectra against pathogenic microorganisms of various, agricultural and horticultural diseases of plants, such as rice sheath blight (Rhizoctonia solani), rice blast (Pyricularia oryzae), cucumber downy mildew (Pseudoperonospora cubensis), cucumber powdery mildew (Sphaerotheca fuliginea), cucumber gray mold (Botrytis cinerea) and Alternaria sooty spot of Chinese mustard (Alternaria brassicicola). In particular, the compounds of this invention exhibit outstanding activity to control rice sheath blight (Rhizoctonia solani). Their fungicidal activities can appear in both the preventive treatment and curative treatment of the plant diseases and moreover are long-lasting. In addition, the new compounds of this invention are of high safety, due to their low toxicity to crops, warm-blooded animals, and a variety of fish and shellfish.

Effects of the fungicidal compounds of this invention are now illustrated by the following Tests.

Test 1: (Test on the preventive effects for cucumber downy mildew, Pseudoperosponora cubensis)

Cucumber seed (variety: Sagami hanjiro) were sown at a rate of 12 seeds each in PVC (polyvinyl chloride) -made pots of 9 cm X 9 cm. The seeds were allowed to grow in a greenhouse for 7 days. A wettable powder containing a test compound and formulated in accordance with the procedure of Example 17 was diluted with water to concentration of 500 ppm of active ingredient, and the aqueous preparation obtained was then applied at a rate of 10 ml per pot to the cucumber seedlings at their cotyledonous stage. After dried in the air, the seedlings were inoculated with a spore suspension of cucumber downy mildew fungi and then placed in a moist chamber at 20-22 °C. On the seventh day after the inoculation, the extent of lesion was rated in accordance with the following standards and equation to estimate the degree of development of disease and the control value.

Extent of disease:

| | |
|---|---|
| Healthy: | No lesion was observed. |
| Slight: | Leaf area infected < 1/3 of the whole leaf area |
| Medium: | Leaf area infected : 2/3 to 1/3 of the whole leaf area |
| Severe: | Leaf area infected < 1/3 of the whole leaf area |

Degree of development of disease (%) =
[{(number of healthy leaves x 0) + (number of slightly-infected leaves x 1) + (number of medium-infected

leaves x 2) + (number of severely-infected leaves x 3)} / 3N] x 100

where N denotes the total number of leave under test.

$$\text{Control value (\%)} = \left(1 - \frac{\text{Degree of development of disease in treated plot}}{\text{Degree of development of disease in untreated plot}}\right) \times 100$$

Test results of rating evaluated in accordance with the following evaluation standard are given in Table 3 below.

Evaluation standards:

- Class A: Control value of greater than 90%.
- Class B: Control value of from 70% and up to 90%.
- Class C: Control value of greater than 40% and up to 70%.
- Class D: Control value of less than 40%.

The same Comparative Chemicals 1,2,3,4,5 and 6, the known compounds as identified hereinafter, were also tested in the same procedure as above, for the comparison purpose.

Table 3

| Compound No. tested | Evaluation of Control value | Compound No. tested | Evaluation of Control value |
|---|---|---|---|
| 1 | B | 42 | A |
| 2 | A | 43 | A |
| 4 | A | 44 | A |
| 11 | B | 45 | A |
| 13 | B | 46 | B |
| 14 | A | 47 | A |
| 16 | B | 48 | A |
| 17 | B | 49 | A |
| 20 | B | 50 | A |
| 21 | A | 51 | A |
| 22 | B | 52 | A |
| 23 | B | 53 | A |
| 24 | A | 54 | A |
| 28 | B | 55 | B |
| 29 | A | 56 | B |
| 30 | A | 58 | A |
| 31 | A | 59 | A |
| 33 | A | 60 | A |
| 35 | A | 62 | B |
| 37 | A | 63 | A |
| 38 | A | 64 | A |
| 40 | A | 65 | A |
| 41 | A | 67 | A |

| Compound No. tested | Evaluation of Control value | Compound No. tested | Evaluation of Control value |
|---|---|---|---|
| 68 | A | 104 | C |
| 69 | A | 105 | B |
| 72 | A | 109 | A |
| 73 | A | 110 | A |
| 74 | A | 111 | A |
| 76 | A | 112 | A |
| 79 | A | 113 | A |
| 83 | A | 114 | A |
| 84 | A | 115 | A |
| 85 | A | 116 | A |
| 86 | B | 117 | A |
| 87 | A | 118 | B |
| 88 | A | 119 | B |
| 93 | A | 120 | B |
| 94 | A | 121 | A |
| 95 | A | 122 | A |
| 96 | A | 124 | A |
| 97 | A | 125 | A |
| 98 | A | 126 | A |
| 99 | A | 128 | B |
| 101 | A | 129 | A |
| 102 | A | 130 | A |
| 103 | A | 131 | B |

| Compound No. tested | Evaluation of Control value | Compound No. tested | Evaluation of Control value |
|---|---|---|---|
| 132 | A | 161 | A |
| 133 | B | 162 | A |
| 134 | A | 163 | A |
| 135 | A | 164 | A |
| 136 | A | 165 | A |
| 137 | A | 166 | A |
| 138 | B | 167 | A |
| 140 | A | 168 | A |
| 141 | A | 172 | A |
| 142 | A | 173 | C |
| 143 | A | 174 | A |
| 144 | A | 175 | A |
| 145 | A | 176 | A |
| 146 | A | 178 | A |
| 147 | A | 185 | A |
| 148 | B | 186 | A |
| 149 | A | 187 | A |
| 152 | A | 188 | A |
| 154 | A | 190 | A |
| 155 | A | 191 | A |
| 156 | A | 194 | C |
| 157 | A | 195 | B |
| 159 | B | 196 | A |

| Compound No. tested | Evaluation of Control value |
|---|---|
| 197 | B |
| 198 | B |
| 201 | C |
| 202 | C |
| 203 | A |
| 208 | A |
| 209 | A |
| 211 | A |
| 212 | A |
| 213 | A |
| 216 | A |
| 217 | A |
| 218 | B |
| 219 | B |
| 220 | A |
| 221 | A |
| 222 | A |
| 223 | A |
| 225 | A |
| 228 | A |
| 229 | A |
| 230 | A |
| 231 | A |

| Compound No. tested | Evaluation of Control value |
|---|---|
| 232 | A |
| 235 | A |
| 236 | A |
| 237 | A |
| 244 | B |
| 245 | A |
| 247 | B |
| 248 | A |
| Comparative Chemical 1 | D |
| Comparative Chemical 2 | D |
| Comparative Chemical 3 | D |
| Comparative Chemical 4 | C |
| Comparative Chemical 5 | D |
| Comparative Chemical 6 | D |

Note: In Table 3,

Comparative Chemical 1:

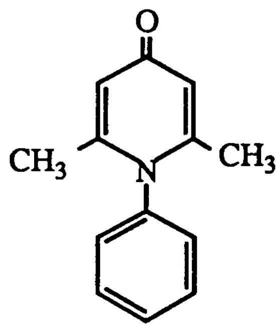

(Disclosed in Japanese Patent Application first publication "Kokai" No. 65871/81)

Comparative Chemical 2:

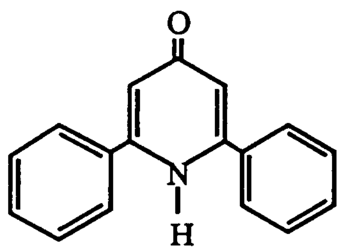

(Disclosed in Japanese Patent Application first publication "Kokai" No. 102504/81)

Comparative Chemical 3:

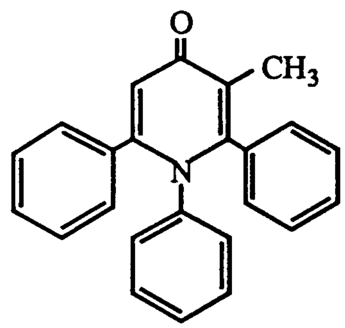

(Disclosed in Japanese Patent Application first publication "Kokai" No. 181204/88)

Comparative Chemical 4:

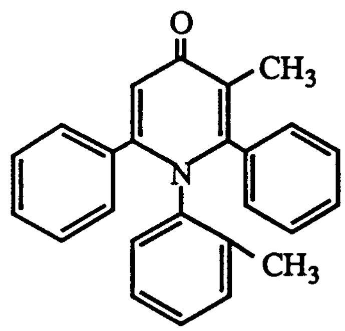

(Disclosed in Japanese Patent Application first publication "Kokai" No. 181204/88)

Comparative Chemical 5:

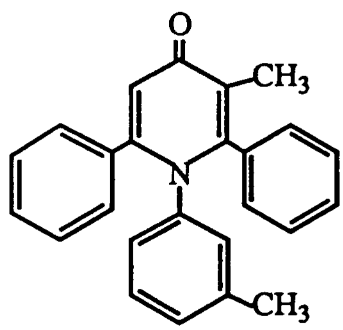

(Disclosed in Japanese Patent Application first publication "Kokai" No. 181204/88)

Comparative Chemical 6:

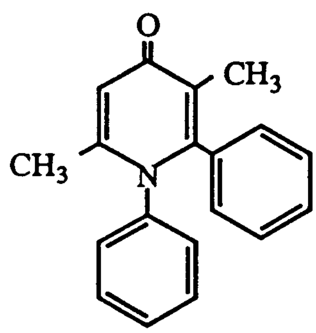

(Disclosed in Japanese journal of "Yakugaku zasshi" 90(5) 613-617(1970))

Test 2: (Test on the preventive effects for cucumber gray mold, Botrytis cinerea)

Cucumber seeds (variety: Sagami Hanjiro) were sown at a rate of 12 seeds each in PVC-made pots of 9 cm x 9 cm. The seeds were allowed to grow for 7 days in a greenhouse. A wettable powder containing a test compound and formulated in accordance with the procedure of Example 2 was diluted with water to a concentration of 500 ppm of the active ingredient, and the aqueous preparation obtained was then sprayed at a rate of 10 ml per pot to the cucumber seedlings at their cotyledonous stage. After dried in the air, the seedlings were inoculated with a suspension of homogenized hypha of cucumber gray mold fungi and then placed in a moist chamber at 20-23 °C. On the fourth day after the inoculation, the overall extent of development of disease in each pot was rated in accordance with the following standard.

Infection index

0: No lesion was observed.
1: Infected area of less than 25% of the total leaf area
2: Infected area of 26-50% of the total leaf area
3: Infected area of 51-75% of the total leaf area

4: Infected area of 76% or more of the total leaf area

Results are shown in Table 4 below. The same comparative chemicals as in Test 1 were also tested in the same way as above, for the comparison purpose.

Table 4

| Compound No. tested | Infection Index | Compound No. tested | Infection Index |
|---|---|---|---|
| 8 | 0 | 74 | 0 |
| 9 | 0 | 75 | 0 |
| 27 | 2 | 78 | 1 |
| 29 | 0 | 79 | 1 |
| 30 | 0 | 83 | 0 |
| 36 | 0 | 84 | 0 |
| 37 | 0 | 85 | 0 |
| 38 | 0 | 86 | 0 |
| 42 | 0 | 87 | 0 |
| 43 | 2 | 88 | 2 |
| 46 | 0 | 91 | 0 |
| 47 | 0 | 92 | 0 |
| 48 | 0 | 99 | 1 |
| 49 | 0 | 100 | 2 |
| 53 | 2 | 101 | 0 |
| 54 | 0 | 102 | 0 |
| 61 | 0 | 103 | 0 |
| 68 | 2 | 104 | 2 |
| 70 | 1 | 105 | 0 |
| 71 | 0 | 106 | 0 |
| 72 | 0 | 108 | 1 |
| 73 | 2 | 109 | 0 |
| 74 | 0 | 110 | 0 |

| Compound No. tested | Infection Index | Compound No. tested | Infection Index |
|---|---|---|---|
| 110 | 0 | 139 | 2 |
| 111 | 2 | 140 | 0 |
| 112 | 2 | 141 | 0 |
| 115 | 2 | 142 | 0 |
| 116 | 0 | 143 | 0 |
| 117 | 0 | 144 | 1 |
| 118 | 1 | 145 | 0 |
| 119 | 2 | 147 | 1 |
| 121 | 0 | 150 | 1 |
| 122 | 1 | 151 | 0 |
| 125 | 0 | 152 | 0 |
| 126 | 0 | 154 | 0 |
| 127 | 0 | 155 | 2 |
| 128 | 0 | 157 | 0 |
| 129 | 0 | 158 | 0 |
| 130 | 0 | 159 | 2 |
| 131 | 0 | 164 | 2 |
| 132 | 0 | 166 | 1 |
| 133 | 2 | 172 | 1 |
| 134 | 2 | 173 | 1 |
| 135 | 2 | 174 | 2 |
| 136 | 2 | 175 | 0 |
| 137 | 1 | 176 | 0 |

| Compound No. tested | Infection Index | Compound No. tested | Infection Index |
|---|---|---|---|
| 178 | 0 | 224 | 2 |
| 189 | 0 | 225 | 1 |
| 190 | 0 | 228 | 0 |
| 191 | 0 | 231 | 1 |
| 192 | 1 | 237 | 1 |
| 193 | 2 | 247 | 2 |
| 194 | 1 | 248 | 0 |
| 195 | 1 | | |
| 196 | 0 | Comparative | |
| 197 | 0 | Chemical 1 | 4 |
| 198 | 0 | Comparative | |
| 201 | 2 | Chemical 2 | 4 |
| 203 | 0 | Comparative | |
| 204 | 0 | Chemical 3 | 2 |
| 206 | 1 | Comparative | |
| 208 | 0 | Chemical 4 | 3 |
| 209 | 1 | Comparative | |
| 210 | 0 | Chemical 5 | 4 |
| 211 | 0 | Comparative | |
| 213 | 1 | Chemical 6 | 4 |
| 217 | 0 | | |
| 218 | 0 | | |
| 223 | 0 | | |

Test 3: (Test on the preventive effects for cucumber powdery mildew, Sphaerotheca fuliginea)

Cucumber seeds (variety: Sagami Hanjiro) were sown at a rate of 12 seeds each in PVC-made pots of 9 cm x 9 cm.The seeds were allowed to germinate and grow for 7 days in a greenhouse. A wettable powder

containing a test compound of Example 17 was diluted with water to a concentration of 500 ppm of the active ingredient, and aqueous preparation obtained was then sprayed at a rate of 10 ml per pot to the cucumber seedlings at their cotyledonous stage. After dried in the air, the seedlings were inoculated with a spore of cucumber mildew fungi and then placed in a greenhouse at 25-30 °C. On the tenth day after the inoculation, the overall extent of development of disease in each pot was rated in accordance with the following standard.

Infection index

0: No lesion was observed.
1: Infected area of less than 25% of the total leaf area.
2: Infected area of 26-50% of the total leaf area
3: Infected area of 51-75% of the total leaf area
4: Infected area of 76% or more of the total leaf area.

Results are shown in Table 5 below. The same comparative chemicals as in Test 1 were also tested in the same way as above, for the comparison purpose.

Table 5

| Compound No. tested | Infection Index | Compound No. tested | Infection Index |
|---|---|---|---|
| 29 | 1 | 74 | 1 |
| 30 | 0 | 75 | 1 |
| 38 | 0 | 78 | 2 |
| 41 | 1 | 79 | 1 |
| 42 | 0 | 85 | 0 |
| 48 | 0 | 86 | 2 |
| 49 | 0 | 87 | 1 |
| 50 | 0 | 88 | 0 |
| 51 | 0 | 90 | 2 |
| 52 | 1 | 92 | 1 |
| 53 | 0 | 93 | 0 |
| 54 | 0 | 96 | 2 |
| 55 | 1 | 97 | 2 |
| 57 | 1 | 105 | 3 |
| 58 | 0 | 111 | 0 |
| 59 | 0 | 115 | 0 |
| 60 | 0 | 116 | 1 |
| 63 | 0 | 117 | 0 |
| 64 | 1 | 124 | 1 |
| 65 | 0 | 126 | 0 |
| 67 | 1 | 128 | 2 |
| 72 | 0 | 129 | 0 |
| 73 | 0 | 130 | 0 |

| Compound No. tested | Infection Index | Compound No. tested | Infection Index |
|---|---|---|---|
| 131 | 0 | 176 | 0 |
| 132 | 0 | 190 | 2 |
| 133 | 1 | 191 | 0 |
| 134 | 1 | 194 | 2 |
| 135 | 0 | 195 | 0 |
| 136 | 0 | 196 | 0 |
| 137 | 0 | 197 | 0 |
| 138 | 2 | 198 | 0 |
| 140 | 3 | 199 | 1 |
| 144 | 1 | 201 | 1 |
| 147 | 1 | 202 | 0 |
| 150 | 2 | 203 | 0 |
| 152 | 1 | 204 | 0 |
| 154 | 0 | 208 | 0 |
| 156 | 0 | 209 | 0 |
| 157 | 0 | 212 | 0 |
| 158 | 0 | 213 | 0 |
| 161 | 0 | 216 | 2 |
| 164 | 1 | 217 | 0 |
| 165 | 1 | 222 | 0 |
| 166 | 0 | 228 | 1 |
| 167 | 0 | 229 | 1 |
| 175 | 0 | 230 | 1 |

| Compound No. tested | Infection Index |
|---|---|
| 231 | 0 |
| 232 | 0 |
| 234 | 1 |
| 235 | 0 |
| 236 | 1 |
| 237 | 0 |
| Comparative Chemical 1 | 4 |
| Comparative Chemical 2 | 3 |
| Comparative Chemical 3 | 4 |
| Comparative Chemical 4 | 4 |
| Comparative Chemical 5 | 4 |
| Comparative Chemical 6 | 4 |

Test 4: (Test on the preventive effects for rice sheath blight, Rhizoctonia solani)

Rice seeds (variety: Kinmaze) were sown at a rate of 15 grains each in unglazed pots having a diameter of 7 cm. The seeds were allowed to grow for 4-5 weeks in a greenhouse. A wettable powder containing a test compound and formulated in accordance with the procedure of Example 17 was diluted with water to a concentration of 500 ppm of the active ingredient, and the aqueous preparation obtained was then sprayed at a rate of 10 ml per pot to the rice seedling at their 5 leaf stage. After dried in the air, the seedlings were inoculated at the basal parts with fungi which had been cultured for 7 days in a rice hull-wheat bran culture

medium, and the inoculated rice plants were then kept in a moist chamber (28 °C). Five days later, the heights of lesions formed on rice leaf sheaths were measured individually. Control value (%) was then evaluated in accordance with the following equation.

$$\text{Control value (\%)} = \left(1 - \frac{\text{Height of lesion in treated plot}}{\text{Height of lesion in untreated plot}}\right) \times 100$$

Test results of rating evaluated in accordance with the following evaluation standard are given in Table 6 below.

Class A: Control value of greater than 90%.
Class B: Control value of from 70% and up to 90%.
Class C: Control value of greater than 40% and up to 70%.
Class D: Control value of less than 40%.

The same comparative chemicals as in Test 1 were also tested in the same way as above, for the comparison purpose.

Table 6

| Compound No. tested | Evaluation of Control value | Compound No. tested | Evaluation of Control value |
|---|---|---|---|
| 1 | A | 26 | A |
| 2 | A | 27 | A |
| 3 | A | 28 | A |
| 4 | A | 29 | A |
| 5 | A | 30 | A |
| 6 | A | 31 | A |
| 7 | A | 32 | B |
| 8 | A | 33 | A |
| 9 | B | 34 | A |
| 10 | A | 35 | A |
| 11 | A | 36 | A |
| 12 | A | 37 | A |
| 13 | A | 38 | A |
| 14 | A | 40 | A |
| 15 | A | 41 | A |
| 16 | A | 42 | A |
| 17 | A | 43 | A |
| 19 | A | 44 | A |
| 20 | A | 45 | A |
| 22 | A | 46 | A |
| 23 | A | 47 | A |
| 24 | A | 48 | A |
| 25 | A | 49 | A |

| Compound No. tested | Evaluation of Control value | Compound No. tested | Evaluation of Control value |
|---|---|---|---|
| 50 | A | 73 | A |
| 51 | A | 74 | A |
| 52 | A | 75 | A |
| 53 | A | 76 | A |
| 54 | A | 77 | B |
| 55 | A | 78 | B |
| 56 | A | 79 | A |
| 57 | A | 80 | A |
| 58 | A | 82 | A |
| 59 | A | 83 | A |
| 60 | A | 84 | A |
| 61 | A | 85 | A |
| 62 | A | 86 | A |
| 63 | B | 87 | A |
| 64 | A | 88 | A |
| 65 | A | 89 | A |
| 66 | A | 90 | A |
| 67 | B | 91 | A |
| 68 | A | 92 | A |
| 69 | B | 93 | A |
| 70 | A | 94 | A |
| 71 | A | 95 | A |
| 72 | A | 96 | A |

| Compound No. tested | Evaluation of Control value | Compound No. tested | Evaluation of Control value |
|---|---|---|---|
| 97 | A | 120 | A |
| 98 | A | 121 | A |
| 99 | A | 122 | A |
| 100 | B | 123 | A |
| 101 | A | 124 | A |
| 102 | A | 125 | A |
| 103 | A | 126 | A |
| 104 | A | 127 | A |
| 105 | A | 128 | A |
| 106 | A | 129 | A |
| 107 | A | 130 | A |
| 108 | A | 131 | A |
| 109 | A | 132 | A |
| 110 | A | 133 | A |
| 111 | A | 134 | A |
| 112 | A | 135 | A |
| 113 | A | 136 | A |
| 114 | A | 137 | A |
| 115 | A | 138 | A |
| 116 | A | 139 | A |
| 117 | A | 140 | A |
| 118 | A | 141 | B |
| 119 | A | 142 | B |

| Compound No. tested | Evaluation of Control value | Compound No. tested | Evaluation of Control value |
|---|---|---|---|
| 143 | B | 167 | B |
| 144 | B | 168 | A |
| 145 | A | 172 | A |
| 146 | A | 173 | A |
| 147 | A | 174 | A |
| 148 | B | 175 | A |
| 149 | A | 176 | A |
| 150 | A | 177 | A |
| 151 | A | 178 | A |
| 152 | A | 189 | A |
| 153 | A | 190 | A |
| 154 | A | 191 | A |
| 155 | A | 192 | A |
| 156 | A | 193 | A |
| 157 | A | 194 | A |
| 158 | A | 195 | A |
| 160 | A | 196 | A |
| 161 | A | 197 | A |
| 162 | A | 198 | A |
| 163 | A | 199 | A |
| 164 | A | 200 | A |
| 165 | A | 201 | A |
| 166 | A | 202 | A |

| Compound No. tested | Evaluation of Control value | Compound No. tested | Evaluation of Control value |
|---|---|---|---|
| 203 | A | 229 | A |
| 204 | A | 230 | A |
| 205 | A | 231 | A |
| 206 | A | 232 | A |
| 207 | A | 233 | B |
| 208 | A | 234 | A |
| 209 | A | 235 | A |
| 210 | A | 236 | A |
| 211 | A | 237 | A |
| 212 | A | 241 | A |
| 213 | A | 242 | A |
| 216 | A | 247 | A |
| 217 | A | 248 | A |
| 218 | A | 21 | A |
| 219 | A | 159 | A |
| 221 | A | Comparative | |
| 222 | A | Chemical 1 | D |
| 223 | A | Comparative | |
| 224 | B | Chemical 2 | D |
| 225 | A | Comparative | |
| 226 | A | Chemical 3 | A |
| 227 | A | Comparative | |
| 228 | A | Chemical 4 | A |

| Compound No. tested | Evaluation of Control value |
|---|---|
| Comparative Chemical 5 | A |
| Comparative Chemical 6 | A |

Test 5: (Test on the preventive effects for Alternaria sooty spot of Chinese mustard, Alternaria brassicicola)

Seeds of Chinese mustard were sown at a rate of 12 seeds each in PVC-made pots of 9 cm x 9 cm. The seeds were allowed to grow for 7 days in a greenhouse. A wettable powder containing test compound and formulated in accordance with the procedure of Example 11 was diluted with water to concentration of 500 ppm of the active ingredient, and the aqueous preparation obtained was then sprayed at a rate of 10 ml per pot to the seedlings of the Chinese mustard at their cotyledon stage. After dried in the air, the seedlings were inoculated with a spore suspension of Alternaria sooty spot fungi and then placed in a moist chamber at 30 °C. On the third day after the inoculation, the average number of lesions per leaf was determined to estimate the control value in accordance with the following equation. Test results of rating evaluated in accordance with the following evaluation standard are given in Table 7 below.

$$\text{Control value (\%)} = \left(1 - \frac{\text{Average number of lesions in treated plot}}{\text{Average number of lesions in untreated plot}}\right) \times 100$$

Evaluation standard:

Class A: Control value of greater than 90%
Class B: Control value of from 70% and up to 90%
Class C: Control value of greater than 40% and up to 70%
Class D: Control value of less than 40%

Table 7

| Compound No. tested | Evaluation of Control value | Compound No. tested | Evaluation of Control value |
|---|---|---|---|
| 8 | A | 87 | A |
| 9 | B | 91 | C |
| 29 | B | 92 | B |
| 30 | A | 93 | A |
| 32 | B | 95 | B |
| 33 | A | 96 | A |
| 34 | B | 98 | B |
| 35 | B | 99 | B |
| 38 | A | 100 | C |
| 41 | B | 101 | C |
| 42 | B | 105 | B |
| 44 | B | 106 | B |
| 45 | B | 108 | B |
| 48 | B | 110 | A |
| 49 | A | 111 | A |
| 52 | B | 112 | A |
| 53 | A | 113 | A |
| 63 | B | 115 | A |
| 73 | B | 116 | B |
| 74 | A | 117 | A |
| 75 | A | 119 | B |
| 85 | B | 120 | A |
| 86 | B | 121 | B |

| Compound No. tested | Evaluation of Control value | Compound No. tested | Evaluation of Control value |
|---|---|---|---|
| 123 | B | 158 | A |
| 124 | A | 159 | A |
| 126 | C | 160 | B |
| 127 | C | 161 | A |
| 129 | A | 163 | B |
| 130 | A | 165 | A |
| 131 | A | 166 | A |
| 132 | A | 167 | A |
| 134 | B | 189 | A |
| 135 | C | 190 | A |
| 136 | B | 191 | A |
| 137 | B | 192 | B |
| 138 | A | 193 | B |
| 139 | B | 194 | B |
| 140 | A | 195 | A |
| 146 | A | 196 | A |
| 147 | A | 197 | A |
| 148 | A | 198 | A |
| 149 | A | 199 | B |
| 152 | A | 200 | C |
| 154 | A | 201 | B |
| 156 | B | 202 | A |
| 157 | A | 203 | A |

| Compound No. tested | Evaluation of Control value |
|---|---|
| 204 | A |
| 205 | B |
| 206 | A |
| 208 | A |
| 209 | A |
| 212 | A |
| 213 | A |
| 216 | A |
| 217 | A |
| 218 | B |
| 223 | B |
| 226 | B |
| 228 | B |
| 229 | A |
| 230 | A |
| 231 | B |
| 232 | B |
| 234 | A |
| 235 | B |
| 236 | B |
| 237 | A |
| 248 | A |

Test 6: (Test on the preventive effects for rice blast, Pyricularia oryzae)

Unhulled rice seeds (variety: Aichi Asahi) were sown at a rate of 20 grains each in white porcelain pots having a diameter of 9 cm. The seeds were allowed to germinate and grow for 3-4 weeks in a greenhouse.

A wettable powder containing a test compounds and formulated in accordance with the procedure of Example 17 was diluted with water to a concentration of 500 ppm of the active ingredient, and the aqueous preparation obtained was then sprayed at a rate of 10 ml per pot to the rice seedlings at their 4 leaf stage. After dried in the air, the seedlings were inoculated with a spore suspension of rice blast fungi and then placed in a moist chamber at 25 °C. On the fifth day after the inoculation, the number of lesions was counted to evaluate the control value.

$$\text{Control value (\%)} = \left(1 - \frac{\text{Number of lesions in treated plot}}{\text{Number of lesions in untreated plot}}\right) \times 100$$

The results obtained are shown in Table 8. The same comparative chemicals as Test 1 were also tested in the same manner as above, for the comparison purpose.

Evaluation standard:

| | |
|---|---|
| Class A: | Control value of greater than 90% |
| Class B: | Control value of from 70% and up to 90% |
| Class C: | Control value of greater than 40% and up to 70% |
| Class D: | Control value of less than 40% |

Table 8

| Compound No. tested | Evaluation of Control value | Compound No. tested | Evaluation of Control value |
|---|---|---|---|
| 2 | B | 42 | A |
| 4 | B | 44 | B |
| 5 | B | 45 | A |
| 7 | A | 47 | B |
| 9 | B | 48 | A |
| 10 | B | 49 | A |
| 13 | B | 50 | A |
| 14 | A | 51 | A |
| 16 | A | 52 | A |
| 19 | A | 53 | A |
| 20 | B | 54 | A |
| 21 | B | 55 | B |
| 22 | B | 56 | A |
| 24 | A | 58 | A |
| 25 | A | 59 | A |
| 29 | B | 60 | A |
| 30 | A | 61 | A |
| 35 | B | 63 | A |
| 36 | B | 64 | A |
| 37 | A | 65 | B |
| 38 | A | 67 | B |
| 40 | B | 68 | A |
| 41 | A | 69 | B |

| Compound No. tested | Evaluation of Control value | Compound No. tested | Evaluation of Control value |
|---|---|---|---|
| 70 | A | 103 | A |
| 71 | A | 104 | A |
| 72 | A | 105 | A |
| 73 | A | 106 | A |
| 74 | B | 107 | A |
| 80 | A | 108 | A |
| 83 | A | 109 | A |
| 84 | A | 110 | A |
| 85 | A | 111 | A |
| 86 | A | 112 | B |
| 87 | A | 114 | B |
| 88 | A | 115 | A |
| 90 | A | 116 | A |
| 92 | A | 117 | A |
| 93 | A | 118 | B |
| 95 | B | 119 | A |
| 96 | B | 121 | A |
| 97 | A | 122 | A |
| 98 | A | 123 | A |
| 99 | A | 124 | A |
| 100 | A | 125 | A |
| 101 | A | 126 | A |
| 102 | A | 128 | A |

| Compound No. tested | Evaluation of Control value | Compound No. tested | Evaluation of Control value |
|---|---|---|---|
| 129 | A | 157 | A |
| 130 | A | 158 | A |
| 131 | A | 159 | A |
| 132 | A | 160 | A |
| 134 | A | 161 | A |
| 135 | A | 162 | A |
| 136 | A | 163 | A |
| 137 | A | 164 | A |
| 138 | B | 165 | A |
| 139 | B | 166 | A |
| 140 | A | 167 | A |
| 145 | B | 168 | A |
| 146 | A | 172 | A |
| 147 | A | 173 | B |
| 148 | A | 178 | B |
| 149 | B | 181 | B |
| 150 | A | 184 | A |
| 151 | A | 185 | A |
| 152 | A | 186 | B |
| 153 | A | 187 | A |
| 154 | A | 188 | A |
| 155 | A | 189 | B |
| 156 | A | 190 | A |

| Compound No. tested | Evaluation of Control value | Compound No. tested | Evaluation of Control value |
|---|---|---|---|
| 191 | A | 219 | A |
| 192 | A | 223 | A |
| 193 | A | 226 | A |
| 194 | A | 228 | A |
| 195 | A | 230 | A |
| 196 | A | 231 | A |
| 197 | A | 232 | B |
| 198 | A | 233 | B |
| 199 | A | 234 | B |
| 201 | A | 236 | B |
| 202 | A | 237 | A |
| 203 | A | 241 | B |
| 204 | A | 242 | B |
| 205 | A | 244 | A |
| 206 | A | 245 | A |
| 207 | A | 246 | A |
| 208 | A | 247 | A |
| 209 | A | 248 | A |
| 211 | B | | |
| 212 | A | Comparative Chemical 1 | D |
| 213 | A | | |
| 216 | A | Comparative Chemical 2 | D |
| 217 | A | | |

Test 7:

The procedures of Tests 1 to 6 were respectively repeated using some compounds of this invention as indicated in Table 9 shown below. The control value (%) for the respective plant disease was evaluated in

the same manner as Tests 1, 4, 5 and 6, or the infection index was evaluated in the same manner as in Tests 2 and 3 above.

The test results obtained are rated in accordance with the following evaluation standards:

Class A: Control value of greater than 90%, or Infection index of 0.
Class B: Control value of from 70% and up to 90%, or Infection index of 1.
Class C: Control value of greater than 40% and up to 70%, or Infection index of 2.
Class D: Control value of less than 50%, or Infection index of 3-4.

The results are summarized in Table 9 below.

Table 9

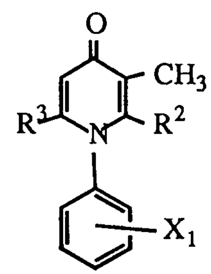

| Compound No. tested | Substituents in tested compound (X)1 | R2 | R3 | Evaluation of Control value: Rice sheath blight | Rice blast | Cucumber downy mildew | Cucumber gray mold | Cucumber powdery mildew | Alternaria sooty spot |
|---|---|---|---|---|---|---|---|---|---|
| 109 | 3,5-$(OCH_3)_2$ | 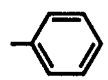 | $-C_3H_7$-n | A | A | A | A | D | A |
| 165 | ” | ” | 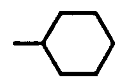 | A | A | A | D | B | B |
| 156 | ” | ” | 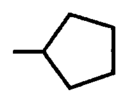 | A | A | A | D | A | A |

Cont'd.........

| | | | | | | | | | |
|---|---|---|---|---|---|---|---|---|---|
| 85 | 2-Cl, 5-OCH3 | 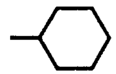 | 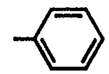 | A | A | A | A | A | C |
| 49 | 3,5-(OCH3)2 | -C4H9-n | ,, | B | B | A | A | A | A |
| 117 | 2-Cl, 5-OCH3 | 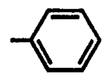 | -C3H7-i | A | A | A | A | A | B |
| 190 | 2-Cl | ,, | 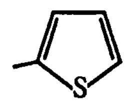 | A | B | A | A | C | A |
| 237 | 3,5-(OCH3)2 | 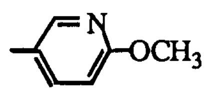 | 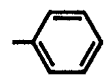 | A | A | A | B | A | B |
| 157 | 2-Cl, 5-OCH3 | 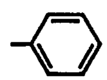 | 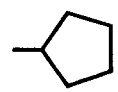 | A | A | A | A | A | A |
| 217 | 3,5-(OCH3)2 | 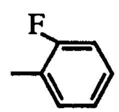 | 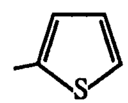 | A | A | A | A | A | A |
| 132 | 2-Cl, 5-OCH3 | 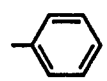 | -C4H9-s | A | A | A | A | A | A |

Cont'd .......

| | | | | | | | | | |
|---|---|---|---|---|---|---|---|---|---|
| 196 | 3,5-$(OCH_3)_2$ | 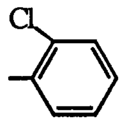 | $-C_4H_9$-s | A | A | A | A | A | A |
| 131 | 2-Cl, 3,5-$(OCH_3)_2$ | 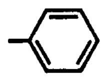 | „ | A | A | B | A | A | A |
| 88 | „ | 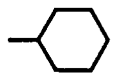 | 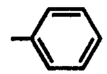 | B | A | A | C | A | A |
| 161 | „ | 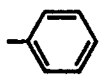 | 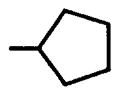 | A | A | A | D | A | A |

## Claims

1. A pyridinone derivative represented by the general formula (I):

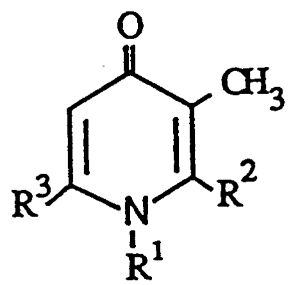

(I)

wherein $R^1$ represents a cycloalkyl group having 3 ~ 6 carbon atoms; a pyridyl group which may be substituted with an alkoxy group having 1 ~ 6 carbon atoms or a halogen atom; a morpholino group; a pyrrolidino group; a piperidino group; a phenyl group which may be substituted with a halogen atom, a lower alkyl group having 1 ~ 6 carbon atoms, a lower alkoxy group having 1 ~ 6 carbon atoms, a trifluoromethyl group or a nitro group; a benzyl group which may be substituted with a halogen atom;

$R^2$ represents a hydrogen atom; an alkyl group having 1 ~ 10 carbon atoms ;

a cycloalkyl group having 3 ~ 6 carbon atoms; a phenyl group which may be substituted with a halogen atom; a naphthyl group; a pyridyl group which may be substituted with a loner alkoxy group having 1 ~ 6 carbon atoms; a thienyl group which may be substituted with a lower alkyl group having 1 ~ 6 carbon atoms; a furyl group which may be substituted with a lower alkyl group having 1 ~ 6 carbon atoms; a pyridin-1-oxide group; an 1,3,5-trimethylpyrazol-4-yl group;

$R^3$ represents a hydrogen atom; an alkyl group having 2 ~ 10 carbon atoms; a cycloalkyl group having 3 ~ 6 carbon atoms; a cycloalkenyl group having 6 carbon atoms; a phenyl group which may be substituted with a halogen atom; a pyridyl group which may be substituted with a lower alkoxy group having 1 ~ 6 carbon atoms; a furyl group which may be substituted with a lower alkyl group having 1 ~ 6 carbon atoms; a naphthyl group; a thienyl group which may be substituted with a lower alkyl group having 1 ~ 6 carbon atoms; or a 3-thienylmethyl group;

with the proviso that $R^1$, $R^2$ and $R^3$ are not an unsubstituted or substituted phenyl group simultaneously.

2. A compound having the formula shown in Claim 1, in which $R^1$ represents an alkyl group having 1 ~ 10 carbon atoms; a cycloalkyl group having 3 ~ 6 carbon atoms; a pyridyl group which may be substituted with a lower alkoxy group having 1 ~ 4 carbon atoms, a lower alkyl group having 1 ~ 4 carbon atoms or a halogen atom; a perhydroazepin-1-yl group; a morpholino group; a pyrrolidino group; a pyrimidyl group which may be substituted with a halogen atom, a furylmethyl group or piperidino group;

$R^2$ represents a phenyl group;

$R^3$ represents a substituted or unsubstituted phenyl group of the formula:

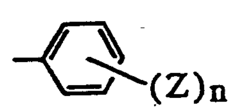

wherein Z is a halogen atom and n is an integer of 0 to 2.

3. A compound as claimed in Claim 1, in which $R^1$ represents a substituted or unsubstituted phenyl group of the formula :

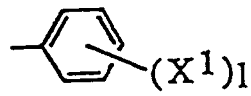

wherein $X^1$ is a halogen atom or a lower alkoxy group having one carbon atom and l is an integer of 0 to 3;

$R^2$ represents a phenyl group, a pyridin-1-oxide group or a furyl group which may be substituted with a lower alkyl group having 1-6 carbon atoms;

$R^3$ represents a phenyl group, a pyridyl group which may be substituted with a lower alkoxy group having 1-6 carbon atoms or a furyl group which may be substituted with a lower alkoxy group having 1-6 carbon atoms.

**4.** A compound as claimed in Claim 1, in which $R^1$ represents a substituted or unsubstituted phenyl group of the formula:

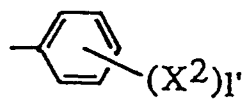

wherein $X^2$ is a halogen atom, a lower alkyl group having 1-6 carbon atoms,a lower alkoxy group having 1-6 carbon atoms,a trifluoromethyl group or a nitro group and l' is an integer of 0 to 3;

$R^2$ represents a substituted or unsubstituted phenyl group of the formula:

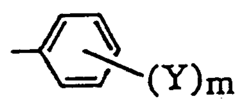

wherein Y represents a halogen atom and m is an integer of 0 to 1;

$R^3$ represents a hydrogen atom or an alkyl group having 2 ~ 7 carbon atoms.

**5.** A compound having the formula shown in Claim 1, in which $R^1$ is a benzyl group which may be substituted with a halogen atom or a substituted or unsubstituted phenyl group of the formula:

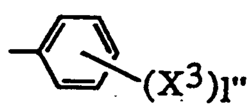

wherein $X^3$ is a halogen atom, a lower alkyl group having 1 ~ 4 carbon atoms, a lower alkoxy group having 1 ~ 4 carbon atoms, a trifluoromethyl group or a nitro group and l'' is an integer of 0 to 3;

$R^2$ represents a hydrogen atom, an alkyl group having 1 ~ 10 carbon atoms which may be substituted with a lower alkoxy group having 1 ~ 4 carbon atoms, a phenyl group which may be substituted with a halogen atom, a pyridyl group which may be substituted with a lower alkoxy group having 1 ~ 4 carbon atoms, a thienyl group which may be substituted with a lower alkyl group having 1 ~ 4 carbon atoms, a cycloalkyl group having 3 ~ 6 carbon atoms, a styryl group or an 1,3,5-trimethylpyrazol-4-yl group;

$R^3$ represents an alkyl group having 2 ~ 10 carbon atoms, a thienyl group which may be substituted with a lower alkyl group having 1 ~ 4 carbon atoms, a phenyl group which may be substituted with a halogen atom, a cycloalkyl group having 3 ~ 6 carbon atoms, a cycloalkenyl group having 6 carbon atoms, a naphthyl group or a 3-thienylmethyl group;

with the proviso that $R^1$, $R^2$ and $R^3$ are not an unsubstituted or substituted phenyl group simultaneously and that $R^2$ is said alkyl group or said cycloalkyl group when $R^3$ is laid alkyl group.

**6.** A compound as claimed in Claim 1, in which at least one of $R^1$, $R^2$ and $R^3$ is a phenyl group.

**7.** A compound as claimed in Claim 6, in which $R^1$ is a mono-, di- or tri-substituted phenyl group.

**8.** A compound as claimed in Claim 7, in which $R^1$ is a halogen- or methoxy-substituted phenyl group.

**9.** A compound as claimed in Claim 6, in which one of $R^2$ and $R^3$ is an alkyl group having 2 ~ 7 carbon atoms and one member selected from the rest of $R^2$ and $R^3$ and $R^1$ is a substituted phenyl group.

**10.** A compound as claimed in Claim 1, in which two for each of $R^1$, $R^2$ and $R^3$ are phenyl groups.

**11.** A compound as claimed in Claim 10, in which $R^1$ is a phenyl group and $R^2$ or $R^3$ is a phenyl group or a substituted phenyl group.

**12.** An agricultural or horticultural fungicidal composition comprising a fungicidally effective amount of a pyridinone derivative represented by the general formula (I):

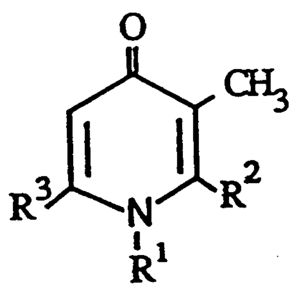

(I)

wherein $R^1$ represents a cycloalkyl group having 3 ~ 6 carbon atoms; a pyridyl group which may be substituted with an alkoxy group having 1 ~ 4 carbon atoms or a halogen atom; a morpholino group; a pyrrolidino group; a piperidino group; a phenyl group which may be substituted with a halogen atom, a lower alkyl group having 1 ~ 4 carbon atoms, a lower alkoxy group having 1 ~ 4 carbon atoms, a trifluoromethyl group or a nitro group; a benzyl group which may be substituted with a halogen atom;

$R^2$ represents a hydrogen atom; an alkyl group having 1 ~ 10 carbon atoms ;

a cycloalkyl group having 3 ~ 6 carbon atoms; a phenyl group which may be substituted with a halogen atom; a naphthyl group; a pyridyl group which may be substituted with a lower alkoxy group having 1 ~ 4 carbon atoms; a thieflyl group which may be substituted with a lower alkyl group having 1 ~ 4 carbon atoms; a furyl group which may be substituted with a lower alkyl group having 1 ~ 4 carbon atoms; a pyridin-1-oxide group; an 1,3,5-trimethylpyrazol-4-yl group; or a styryl group;

$R^3$ represents a hydrogen atom; an alkyl group having 2 ~ 10 carbon atoms; a cycloalkyl group having 3 ~ 6 carbon atoms; a cycloalkenyl group having 6 carbon atoms; a phenyl group which may be substituted with a halogen atom; a pyridyl group which may be substituted with a lower alkoxy group having 1 ~ 4 carbon atoms; a furyl group which may be substituted with a lower alkyl group having 1 ~ 4 carbon atoms; a naphthyl group; a thienyl group which may be substituted with a lower alkyl group having 1 ~ 4 carbon atoms; or a 3-thienylmethyl group;

with the proviso that $R^1$, $R^2$ and $R^3$ are not an unsubstituted or substituted phenyl group simultaneously; and an agriculturally or horticulturally acceptable carrier.

**13.** An agricultural or horticultural fungicidal composition comprising a fungicidally effective amount of a pyridinone derivative having the formula shown in claim 12, wherein $R_1$, $R_2$ and $R_3$ have meanings according to any one of the patent claims 2 to 11.

**14.** A process for preparing a pyridinone derivative as defined in any one of the claims 1 to 11, which comprises reacting a pentanetrione compound represented by the general formula (II):

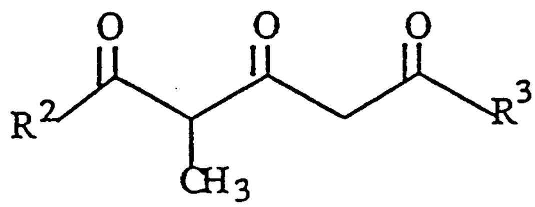

(II)

wherein $R^2$ and $R^3$ have the same meanings as defined above, with an amine compound represented by the general formula (III):

$R^1$-$NH_2$ (III)

wherein $R^1$ has the same meanings as defined above.

**Patentansprüche**

1. Pyridinonderivat, dargestellt durch die allgemeine Formel (I),

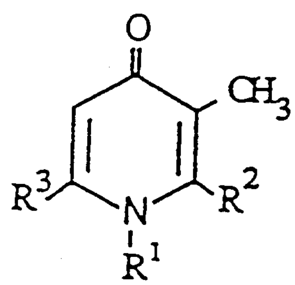

worin $R^1$ eine Cycloalkylgruppe mit 3 bis 6 Kohlenstoffatomen; eine Pyridylgrupe, die mit einer Alkoxygruppe mit 1 bis 6 Kohlenstoffatomen oder einem Halogenatom substituiert sein kann; eine Morpholinogruppe; eine Pyrrolidinogruppe; eine Piperidinogruppe; eine Phenylgruppe, die mit einem Halogenatom, einer Niedrigalkylgruppe mit 1 - 6 Kohlenstoffatomen, einer Niedrigalkoxygruppe mit 1 - 6 Kohlenstoffatomen, einer Trifluoromethylgruppe oder einer Nitrogruppe substituiert sein kann; eine Benzylgruppe ist, die mit einem Halogenatom substituiert sein kann;
worin $R^2$ ein Wasserstoffatom; eine Alkylgruppe mit 1 - 10 Kohlenstoffatomen; eine Cycloalkylgruppe mit 3 bis 6 Kohlenstoffatomen; eine Phenylgruppe, die mit einem Halogenatom substituiert sein kann; eine Naphthylgruppe; eine Pyridylgruppe, die mit einer Niedrigalkoxygruppe mit 1 bis 6 Kohlenstoffatomen substituiert sein kann, eine Thienylgruppe, die mit einer Niedrigalkylgruppe mit 1 bis 6 Kohlenstoffatomen substituiert sein kann, eine Furylgruppe, die mit einer Niedrigalkylgruppe mit 1 bis 6 Kohlenstoffatomen substituiert sein kann; einer Pyridin-1-oxidgruppe; einer 1,3,5-Trimethylpyrazol-4-ylgruppe ist;
worin $R^3$ ein Wasserstoffatom; eine Alkylgruppe mit 2 bis 10 Kohlenstoffatomen; eine Cycloalkylgruppe mit 3 - 6 Kohlenstoffatomen; eine Cycloalkenylgruppe mit 6 Kohlenstoffatomen; eine Phenylgruppe, die mit einem Halogenatom substituiert sein kann; eine Pyridylgruppe, die mit einer Niedrigalkoxygruppe mit 1 - 6 Kohlenstoffatomen oxidiert sein kann, eine Furylgruppe, die mit einer Niedrigalkylgruppe mit 1 - 6 Kohlenstoffatomen substituiert sein kann; eine Naphthylgruppe; eine Thienylgruppe, die mit einer Niedrigalkylgruppe mit 1 - 6 Kohlenstoffatomen substituiert sein kann; oder einer 3-Thienylmethylgruppe ist;
mit dem Vorbehalt, daß $R^1$, $R^2$ und $R^3$ nicht gleichzeitig eine unsubstitueirte oder substituierte Phenylgruppe sind.

2. Verbindung mit der Formel, die in Anspruch 1 gezeigt ist, worin $R^1$ eine Alkylgruppe mit 1 - 10 Kohlenstoffatomen; eine Cycloalkylgruppe mit 3 - 6 Kohlenstoffatomen; eine Pyridylgruppe, die mit einer Niedrigalkoxygruppe mit 1 - 4 Kohlenstoffatomen, einer Niedrigalkylgruppe mit 1 - 4 Kohlenstoffatomen oder einem Halogenatom substituiert sein kann; eine Perhydroazepin-1-yl-Gruppe; eine Morpholinogruppe; eine Pyrrolidinogruppe; eine Pyrimidylgruppe, die mit einem Halogenatom substituiert

sein kann, eine Furylmethylgruppe oder eine Piperidinogruppe ist;
worin $R^2$ eine Phenylgruppe ist,
worin $R^3$ eine substituierte oder unsubstituierte Phenylgruppe der Formel ist:

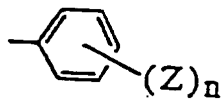

worin Z ein Halogenatom und n eine ganze Zahl von 0 bis 2 ist.

**3.** Verbindung nach Anspruch 1, worin $R^1$ eine substituierte oder unsubstitierte Phenylgruppe der Formel ist:

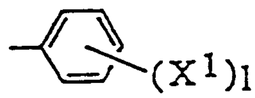

worin $X^1$ ein Halogenatom oder eine Niedrigalkoxygruppe mit einem Kohlenstoffatom ist, und worin l eine ganze Zahl von 0 bis 3 ist;
worin $R^2$ eine Phenylgruppe, eine Pyridin-1-oxidgruppe oder eine Furylgruppe ist, die mit einer Niedrigalkylgruppe mit 1 - 6 Kohlenstoffatomen substituiert sein kann;
worin $R^3$ eine Phenylgruppe, eine Pyridylgruppe, die mit einer Niedrigalkoxygruppe mit 1 - 6 Kohlenstoffatomen substituiert sein kann, oder eine Furylgruppe ist, die mit einer Niedrigalkoxygruppe mit 1 - 6 Kohlenstoffatomen substituiert sein kann.

**4.** Verbindung nach Anspruch 1,
worin $R^1$ eine substituierte oder unsubstituierte Phenylgruppe der Formel ist,

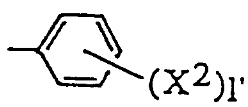

worin $X^2$ ein Halogenatom, eine Niedrigalkylgruppe mit 1 - 6 Kohlenstoffatomen, eine Niedrigalkoxygruppe mit 1 - 6 Kohlenstoffatomen, eine Trifluoromethylgruppe oder eine Nitrogruppe ist, und worin l' eine ganze Zahl von 0 bis 3 ist;
worin $R^2$ eine substituierte oder unsubstituierte Phenylgruppe der Formel ist:

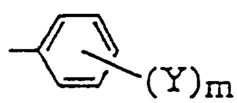

worin Y ein Halogenatom ist und worin m eine ganze Zahl von 0 bis 1 ist;
worin $R^3$ ein Wasserstoffatom oder eine Alkylgruppe mit 2 - 7 Kohlenstoffatomen ist.

**5.** Verbindung mit der Formel, die in Anspruch 1 gezeigt ist, worin $R^1$ eine Benzylgruppe, die mit einem Halogenatom substituiert sein kann, oder eine substituierte oder unsubstituierte Phenylgruppe der Formel ist:

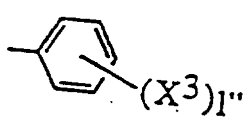

worin $X^3$ ein Halogenatom, eine Niedrigalkylgruppe mit 1 - 4 Kohlenstoffatomen, eine Niedrigalkoxygruppe mit 1 - 4 Kohlenstoffatomen, eine Trifluoromethylgruppe oder eine Nitrogruppe ist, und worin l'' eine ganze Zahl von 0 bis 3 ist;
worin $R^2$ ein Wasserstoffatom, eine Alkylgrupe mit 1 bis 10 Kohlenstoffatomen, die mit einer Niedrigalkoxygruppe mit 1 bis 4 Kohlenstoffatomen substituiert sein kann, eine Phenylgruppe, die mit einem Halogenatom substituiert sein kann, eine Pyridylgruppe, die mit einer Niedrigalkoxygruppe mit 1 - 4 Kohlenstoffatomen substituiert sein kann, eine Thienylgruppe, die mit einer Niedrigalkylgrupe mit 1- 4 Kohlenstoffatomen substituiert sein kann, eine Cycloalkylgruppe mit 3 - 6 Kohlenstoffatomen, eine Styrylgruppe oder eine 1,3,5-Trimethylpyrazol-4-yl Gruppe ist;
worin $R^3$ eine Alkylgruppe mit 2 - 10 Kohlenstoffatomen; eine Thienylgruppe, die mit einer Niedrigalkylgruppe mit 1 - 4 Kohlenstoffatomen substituiert sein kann, eine Phenylgruppe, die mit einem Halogenatom substituiert sein kann, eine Cycloalkylgruppe mit 3 - 6 Kohlenstoffatomen, eine Cycloalkenylgruppe mit 6 Kohlenstoffatomen, eine Naphthylgruppe oder eine 3-Thienylmethylgruppe ist;
mit dem Vorbehalt, daß $R^1$, $R^2$ und $R^3$ nicht gleichzeitig eine unsubstituierte oder substituierte Phenylgruppe sind, und daß $R^2$ die Alkylgruppe oder die Cycloalkylgurppe ist, wenn $R^3$ die Alkylgruppe ist.

6. Verbindung nach Anspruch 1, worin zumindest eines von $R^1$, $R^2$ und $R^3$ eine Phenylgruppe ist.

7. Verbindung nach Anspruch 6, worin $R^1$ eine mono-, di- oder tri-substituierte Phenylgruppe ist.

8. Verbindung nach Anspruch 7, worin $R^1$ eine halogen- oder methoxysubstituierte Phenylgruppe ist.

9. Verbindung nach Anspruch 6, worin eines von $R^2$ und $R^3$ eine Alkylgruppe mit 2 bis 7 Kohlenstoffatomen ist und ein Teil, ausgewählt aus dem Rest von $R^2$ und $R^3$ und $R^1$, eine substituierte Phenylgruppe ist.

10. Verbindung nach Anspruch 1, worin zwei von $R^1$, $R^2$ und $R^3$ Phenylgruppen sind.

11. Verbindung nach Anspruch 10, worin $R^1$ eine Phenylgruppe ist, und worin $R^2$ und $R^3$ eine Phenylgruppe oder eine substituierte Phenylgruppe ist.

12. Fungizide Zusammensetzung für die Landwirtschaft oder den Gartenbau, umfassend eine fungizid wirksame Menge eines Pyridinonderivates, dargestellt durch die allgemeine Formel (I)

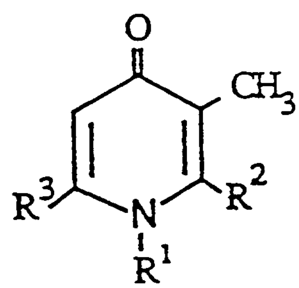

worin $R^1$ eine cycloalkylgruppe mit 3 bis 6 Kohlenstoffatomen; eine Pyridylgrupe, die mit einer Alkoxygruppe mit 1 bis 4 Kohlenstoffatomen oder einem Halogenatom substituiert sein kann; eine Morpholinogruppe; eine Pyrrolidinogruppe; eine Piperidinogruppe; eine Phenylgruppe, die mit einem Halogenatom, einer Niedrigalkylgruppe mit 1 - 4 Kohlenstoffatomen, einer Niedrigalkoxygruppe mit 1 - 4 Kohlenstoffatomen, einer Trifluoromethylgruppe oder einer Nitrogruppe substituiert sein kann; eine Benzylgruppe ist, die mit einem Halogenatom substituiert sein kann;
worin $R^2$ ein Wasserstoffatom; eine Alkylgruppe mit 1 - 10 Kohlenstoffatomen; eine Cycloalkylgruppe mit 3 bis 6 Kohlenstoffatomen; eine Phenylgruppe, die mit einem Halogenatom substituiert sein kann; eine Naphthylgruppe; eine Pyridylgruppe, die mit einer Niedrigalkoxygruppe mit 1 bis 6 Kohlenstoffatomen substituiert sein kann; eine Thienylgruppe, die mit einer Niedrigalkylgruppe mit 1 bis 6 Kohlenstoffatomen substituiert sein kann; eine Furylgruppe, die mit einer Niedrigalkylgruppe mit 1 bis 4 Kohlenstoffatomen substituiert sein kann; eine Pyridin-1-oxidgruppe; eine 1,3,5-Trimethylpyrazol-4-ylgruppe ist.

worin $R^3$ ein Wasserstoffatom; eine Alkylgruppe mit 2 bis 10 Kohlenstoffatomen; eine Cycloalkylgruppe mit 3 - 6 Kohlenstoffatomen; eine Cycloalkenylgruppe mit 6 Kohlenstoffatomen; eine Phenylgruppe, die mit einem Halogenatom substituiert sein kann; eine Pyridylgruppe, die mit einer Niedrigalkoxygruppe mit 1 - 4 Kohlenstoffatomen oxidiert sein kann; eine Furylgruppe, die mit einer Niedrigalkylgruppe mit 1 - 4 Kohlenstoffatomen substituiert sein kann; eine Naphthylgruppe; eine Thienylgruppe, die mit einer Niedrigalkylgruppe mit 1 - 4 Kohlenstoffatomen substituiert sein kann; oder eine 3-Thienylmethylgruppe ist;

mit dem Vorbehalt, daß $R^1$, $R^2$ und $R^3$ nicht gleichzeitig eine unsubstituierte oder substituierte Phenylgruppe sind;

und einen landwirtschaftlich oder gartenbaulich akzeptablen Träger.

**13.** Landwirtschaftlich oder gartenbaulich fungizide Zusammensetzung, umfassend eine fungizid effektive Menge eines Pyridinonderivates mit der in Anspruch 12 gezeigten Formel, worin $R^1$, $R^2$ und $R^3$ Bedeutungen entsprechend einem der Patentansprüche 2 bis 11 haben.

**14.** Verfahren zur Herstellung eines Pyridinonderivates nach einem der Ansprüche 1 bis 11, umfassend die Reaktion einer Pentantrionverbindung, dargestellt durch die allgemeine Formel (II):

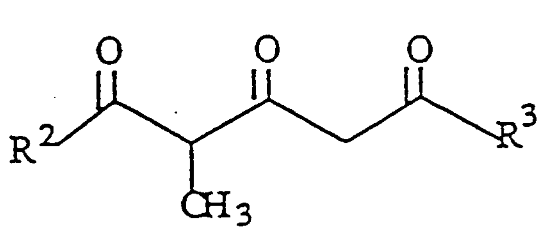

(II)

worin $R^2$ und $R^3$ die gleichen Bedeutungen wie oben aufweisen, mit einer Aminverbindung, dargestellt durch die allgemeine Formel (III):

$R^1$-$NH_2$ (III)

worin $R^1$ die gleichen Bedeutungen wie oben definiert hat.

## Revendications

**1.** Dérivé pyridinone, représenté par la formule générale (I) :

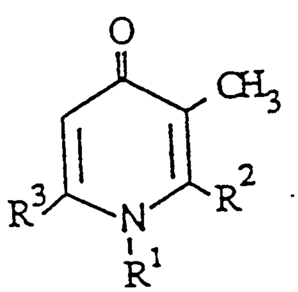

(I)

dans laquelle $R^1$ représente un groupe cycloalkyle comportant 3-6 atomes de carbone; un groupe pyridyle qui peut être substitué par un groupe alcoxy comportant 1-6 atomes de carbone ou un atome d'halogène; un groupe morpholino; un groupe pyrrolidino; un groupe pipéridino; un groupe phényle, qui peut être substitué par un atome d'halogène, un groupe alkyle inférieur comportant 1-6 atomes de carbone, un groupe alcoxy inférieur comportant 1-6 atones de carbone, un groupe trifluorométhyle ou un groupe nitro; un groupe benzyle, qui peut être substitué par un atome d'halogène;

$R^2$ représente un atome d'hydrogène; un groupe alkyle comportant 1-10 atomes de carbone; un groupe cycloalkyle comportant 3-6 atomes de carbone; un groupe phényle, qui peut être substitué par un atome d'halogène; un groupe naphtyle; un groupe pyridyle, qui peut être substitué par un groupe

alcoxy inférieur comportant 1-6 atomes de carbone; un groupe thiényle, qui peut être substitué par un groupe alkyle inférieur comportant 1-6 atomes de carbone; un groupe furyle, qui peut être substitué par un groupe alkyle inférieur comportant 1-6 atomes de carbone; un groupe pyridine-1-oxyde; un groupe 1,3,5-triméthylpyrazole-4-yle;

$R^3$ représente un atome d'hydrogène; un groupe alkyle comportant 2-10 atomes de carbone; un groupe cycloalkyle comportant 3-6 atomes de carbone; un groupe cycloalcényle comportant 6 atomes de carbone; un groupe phényle qui peut être substitué par un atome d'halogène; un groupe pyridyle qui peut être substitué par un groupe alcoxy inférieur comportant 1-6 atomes de carbone; un groupe furyle qui peut être substitué par un groupe alkyle inférieur comportant 1-6 atomes de carbone; un groupe naphtyle; un groupe thiényle qui peut être substitué par un groupe alkyle inférieur comportant 1-6 atomes de carbone; ou un groupe 3-thiénylméthyle;

à condition que $R^1$, $R^2$ et $R^3$ ne soient pas simultanément des groupes phényle non substitués ou substitués.

**2.** Composé de formule indiquée dans la revendication 1, dans laquelle $R^1$ représente un groupe alkyle comportant 1-10 atomes de carbone; un groupe cycloalkyle comportant 3-6 atomes de carbone; un groupe pyridyle qui peut être substitué par un groupe alcoxy inférieur comportant 1-4 atomes de carbone, un groupe alkyle inférieur comportant 1-4 atomes de carbone ou par un atome d'halogène; un groupe perhydroazépine-1-yle; un groupe morpholino, un groupe pyrrolidino; un groupe pyrimidyle qui peut être substitué par un atome d'halogène, un groupe furylméthyle ou un groupe pipéridino;

$R^2$ représente un groupe phényle;

$R^3$ représente un groupe phényle substitué ou non substitué de formule

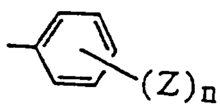

dans laquelle Z est un atome d'halogène et n est un nombre entier de 0 à 2.

**3.** Composé selon la revendication 1, dans lequel $R^1$ représente un groupe phényle substitué ou non substitué de formule

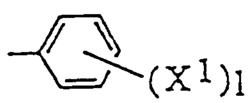

dans laquelle $X^1$ est un atome d'halogène ou un groupe alcoxy inférieur comportant un atome de carbone et l est un nombre entier de 0 à 3;

$R^2$ représente un groupe phényle, un groupe pyridine-1-oxyde ou un groupe furyle qui peut être substitué par un groupe alkyle inférieur comportant 1-6 atomes de carbone;

$R^3$ représente un groupe phényle, un groupe pyridyle, qui peut être substitué par un groupe alcoxy inférieur comportant 1-6 atomes de carbone, ou un groupe furyle qui peut être substitué par un groupe alcoxy inférieur comportant 1-6 atomes de carbone.

**4.** Composé selon la revendication 1, dans lequel $R^1$ représente un groupe phényle substitué ou non substitué de formule :

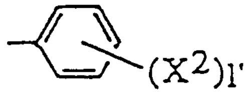

dans laquelle $X^2$ est un atome d'halogène, un groupe alkyle inférieur comportant 1-6 atomes de carbone, un groupe alcoxy inférieur comportant 1-6 atomes de carbone, un groupe trifluorométhyle ou un groupe nitro, et l' est un nombre entier de 0 à 3;

$R^2$ représente un groupe phényle substitué ou non substitué de formule :

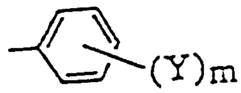

dans laquelle Y représente un atome d'halogène et m est un nombre entier de 0 à 1;

$R^3$ représente un atome d'hydrogène ou un groupe alkyle comportant 2-7 atomes de carbone.

**5.** Composé de formule indiquée dans la revendication 1, dans laquelle $R^1$ représente un groupe benzyle, qui peut être substitué par un atome d'halogène, ou un groupe phényle substitué ou non substitué de formule

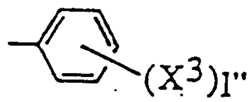

dans laquelle $X^3$ est un atome d'halogène, un groupe alkyle inférieur comportant 1-4 atomes de carbone, un groupe alcoxy inférieur comportant 1-4 atomes de carbone, un groupe trifluorométhyle ou un groupe nitro, et l'' est un nombre entier de 0 à 3;

$R^2$ représente un atome d'hydrogène, un groupe alkyle comportant 1-10 atomes de carbone, qui peut être substitué par un groupe alcoxy inférieur comportant 1-4 atomes de carbone, un groupe phényle qui peut être substitué par un atome d'halogène, un groupe pyridyle qui peut être substitué par un groupe alcoxy inférieur comportant 1-4 atomes de carbone, un groupe thiényle qui peut être substitué par un groupe alkyle inférieur comportant 1-4 atomes de carbone, un groupe cycloalkyle comportant 3-6 atomes de carbone, un groupe styryle ou un groupe 1,3,5-triméthylpyrazole-4-yle;

$R^3$ représente un groupe alkyle comportant 2-10 atomes de carbone, un groupe thiényle, qui peut être substitué par un groupe alkyle inférieur comportant 1-4 atomes de carbone, un groupe phényle qui peut être substitué par un atome d'halogène, un groupe cycloalkyle comportant 3-6 atomes de carbone, un groupe cycloalcényle comportant 6 atomes de carbone, un groupe naphtyle ou un groupe 3-thiénylméthyle;

à condition que $R^1$, $R^2$ et $R^3$ ne soient pas simultanément des groupes phényle non substitués ou substitués et que $R^2$ soit ledit groupe alkyle ou ledit groupe cycloalkyle lorsque $R^3$ est ledit groupe alkyle.

**6.** Composé selon la revendication 1, dans lequel l'un au moins des radicaux $R^1$, $R^2$ et $R^3$ est un groupe phényle.

**7.** Composé selon la revendication 6, dans lequel $R^1$ est un groupe phényle mono-, di- ou tri-substitué.

**8.** Composé selon la revendication 7, dans lequel $R^1$ est un groupe phényle halogéno- ou méthoxy-substitué.

**9.** Composé selon la revendication 6, dans lequel l'un des radicaux $R^2$ et $R^3$ est un groupe alkyle comportant 2-7 atomes de carbone et un des radicaux choisis dans le reste de $R^2$ et $R^3$ et $R^1$ est un groupe phényle substitué.

**10.** Composé selon la revendication 1, dans lequel deux des radicaux $R^1$, $R^2$ et $R^3$ sont des groupes phényle.

**11.** Composé selon la revendication 10, dans lequel $R^1$ est un groupe phényle et $R^2$ ou $R^3$ est un groupe phényle ou un groupe phényle substitué.

**12.** Composition fongicide agricole ou horticole, comprenant une quantité efficace en tant que fongicide d'un dérivé pyridinone représenté par la formule générale (I) :

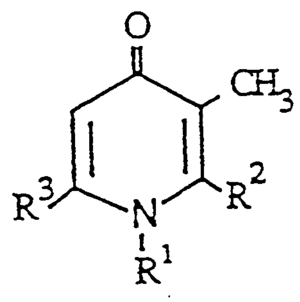

(I)

dans laquelle $R^1$ représente un groupe cycloalkyle comportant 3-6 atomes de carbone; un groupe pyridyle qui peut être substitué par un groupe alcoxy comportant 1-4 atomes de carbone ou un atome d'halogène; un groupe morpholino; un groupe pyrrolidino; un groupe pipéridino; un groupe phényle, qui peut être substitué par un atome d'halogène, un groupe alkyle inférieur comportant 1-4 atomes de carbone, un groupe alcoxy inférieur comportant 1-4 atomes de carbone, un groupe trifluorométhyle ou un groupe nitro; un groupe benzyle, qui peut être substitué par un atome d'halogène;

$R^2$ représente un atome d'hydrogène; un groupe alkyle comportant 1-10 atomes de carbone; un groupe cycloalkyle comportant 3-6 atomes de carbone; un groupe phényle, qui peut être substitué par un atome d'halogène; un groupe naphtyle; un groupe pyridyle, qui peut être substitué par un groupe alcoxy inférieur comportant 1-4 atomes de carbone; un groupe thiényle, qui peut être substitué par un groupe alkyle inférieur comportant 1-4 atomes de carbone; un groupe furyle, qui peut être substitué par un groupe alkyle inférieur comportant 1-4 atomes de carbone; un groupe pyridine-1-oxyde; un groupe 1,3,5-triméthylpyrazole-4-yle; ou un groupe styryle;

$R^3$ représente un atome d'hydrogène; un groupe alkyle comportant 2-10 atomes de carbone; un groupe cycloalkyle comportant 3-6 atomes de carbone; un groupe cycloalcényle comportant 6 atomes de carbone; un groupe phényle qui peut être substitué par un atome d'halogène; un groupe pyridyle qui peut être substitué par un groupe alcoxy inférieur comportant 1-4 atomes de carbone; un groupe furyle qui peut être substitué par un groupe alkyle inférieur comportant 1-4 atomes de carbone; un groupe naphtyle; un groupe thiényle qui peut être substitué par un groupe alkyle inférieur comportant 1-4 atomes de carbone; ou un groupe 3-thiénylméthyle;

à condition que $R^1$, $R^2$ et $R^3$ ne soient pas simultanément des groupes phényle non substitués ou substitués, en même temps qu'un véhicule acceptable en agriculture et en horticulture.

**13.** Composition fongicide agricole ou horticole, comprenant une quantité efficace en tant que fongicide d'un dérivé pyridinone dont la formule est représentée dans la revendication 12, dans laquelle $R^1$, $R^2$ et $R^3$ ont les significations définies dans l'une quelconque des revendications 2 à 11.

**14.** Procédé de préparation d'un dérivé pyridinone selon l'une quelconque des revendications 1 à 11, qui comprend la réaction d'un composé pentanetrione représenté par la formule générale (II) :

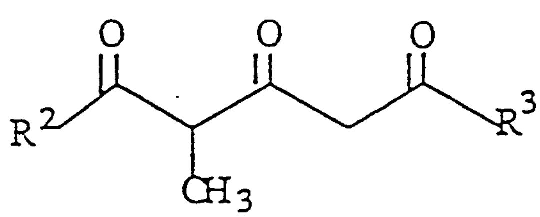

(II)

dans laquelle $R^2$ et $R^3$ ont les mêmes significations que ci-dessus, ou un de ses tautomères, avec un dérivé amine représenté par la formule générale (III) :

$R^1$-$NH_2$ (III)

dans laquelle $R^1$ a la même signification que ci-dessus.